# EUROPÄISCHE PATENTSCHRIFT

(11) **EP 2 807 979 B1**
(45) Veröffentlichungstag und Bekanntmachung des Hinweises auf die Patenterteilung: **11.10.2017**
(21) Anmeldenummer: 14167170.1
(22) Anmeldetag: 06.05.2014
(51) Int. Cl.: A61B 10/00, A61B 10/02, A61B 5/145, G01N 33/543

(54) **Detektionsvorrichtung zur in vivo und/oder in vitro Anreicherung von Probenmaterial**
Detection device for in-vivo and/or in-vitro enrichment of sample material
Dispositif de détection destiné à améliorer in vivo et/ou in vitro un matériau d'échantillon

(30) Priorität: 31.05.2013 DE 102013210204
(43) Veröffentlichungstag der Anmeldung: 03.12.2014
(73) Patentinhaber: GILUPI GmbH, 14473 Potsdam (DE)
(72) Erfinder: Niestroj-Pahl, Robert, 10249 Berlin (DE); Lücke, Klaus, 14542 Werder (DE); Bollmann, Andreas, 12685 Berlin (DE)
(74) Vertreter: Grünecker Patent- und Rechtsanwälte PartG mbB

(56) Entgegenhaltungen:
- WO-A1-2010/025719
- WO-A1-2010/145824
- WO-A1-2011/047671
- WO-A1-2011/113584
- FLORES L M ET AL: "Improving the yield of circulating tumour cells facilitates molecular characterisation and recognition of discordant HER2 amplification in breast cancer", BRITISH JOURNAL OF CANCER, NATURE PUBLISHING GROUP, GB , Bd. 102, Nr. 10 1. Mai 2010 (2010-05-01), Seiten 1495-1502, XP002671705, ISSN: 0007-0920, DOI: 10.1038/SJ.BJC.6605676 Gefunden im Internet: URL:http://www.nature.com/bjc/journal/v102 /n10/full/6605676a.html [gefunden am 2010-05-11]
- SAUCEDO-ZENI N ET AL: "A novel method for the in vivo isolation of circulating tumor cells from peripheral blood of cancer patients using a functionalized and structured medical wire", INTERNATIONAL JOURNAL OF ONCOLOGY, Bd. 41, 16. Juli 2012 (2012-07-16), Seiten 1241-1250, XP002731287, DOI: 10.3892/ijo.2012.1557

## Beschreibung

Die Erfindung betrifft eine Detektionsvorrichtung zur in vivo und/oder in vitro Anreicherung von Probenmaterial, umfassend eine mit Detektionsrezeptoren bestückte Funktionsfläche.

Viele Zelltypen, Moleküle, Tumor- und Biomarker sind in Körperflüssigkeiten zwar vorhanden, können aber oft wegen ihrer niedrigen Konzentration nicht effizient genug durch die herkömmlichen Methoden der Anreicherung gewonnen werden, um anschließend in etablierten diagnostischen Verfahren der klinischen Chemie, Pathologie oder Zytologie genutzt werden zu können.

Zum Beispiel ist das Anreichern von speziellen Zellen, insbesondere zirkulierenden Tumorzellen, aus einer Blutprobe außerhalb des Körpers eines Patienten (in vitro) mittels kommerziell erhältlicher paramagnetischer Nanopartikel und/oder Dichtegradientenzentrifugation möglich, jedoch in nur sehr begrenzter Anzahl und mit dem Nachteil, dass die Nanopartikel an oder in der Zelle binden und diese damit schädigen bzw. die Diagnostik erschweren können. Eine dieser kommerziellen Methoden spiegelt sich in einem Test wieder, in dem z.B. zirkulierende Tumorzellen aus 7,5 ml Blutvolumen mittels paramagnetischer Nanopartikel angereichert werden, um dann Aussagen über den Krankheitsverlauf machen zu können.

Der limitierende Faktor dieser Methode ist das gewonnene Probevolumen, welches beim Anwenden einer Detektionsvorrichtung zur Anreicherung von Probenmaterial innerhalb des Körpers eines Patienten (in vivo), z. B. eines funktionalisierten Katheter, um ein Vielfaches höher ist. Gefäßkatheter für die Anwendung medizinischer Interventionen sind meist zylinderförmig konstruiert. Der Vorteil dieser Form ist der relativ geringe Reibungswiderstand. Dennoch besteht bei dieser Form die Gefahr, dass der Blutstrom in kleineren Blutgefäßen verengt wird und dies zur Ausbildung einer Thrombose führt.

Aus dem Stand der Technik in der WO 2010/145824 A1 ist ferner eine Detektionsvorrichtung zur in vivo und/oder in vitro Anreicherung von Probenmaterial bekannt, bei der eine mit Detektionsrezeptoren bestückte Funktionsfläche eine dreidimensionale Struktur mit einander zugewandten Funktionsabschnitten aufweist, die von einer Probenflüssigkeit durchsetzbare Zwischenräume bilden, wodurch die Gefahr von Thrombosen verringert wird. Gemäß der WO 2010/145824 A1 können die einzelnen Funktionsabschnitte der Funktionsfläche mit chemisch identischen oder chemischen verschiedenen Detektionsrezeptoren bestückt sein. Bei Bestückung mit unterschiedlichen Detektionsrezeptoren kann eine derartige Detektionsvorrichtung beispielsweise für die Diagnose unterschiedlicher Krankheiten eingesetzt werden.

Des Weiteren ist aus dem Stand der Technik in der WO 2011/047671 A1 ein magnetisches Biopsie-Instrument und dessen Verwendung zur Anreicherung von spezifischem Probenmaterial aus dem Körper bekannt. Das Biopsie-Instrument besteht mindestens aus einem Führungselement, einem magnetischen Element und einem Stabilisierungselement. Das Führungselement weist einen distalen und einen proximalen Bereich auf, wobei der distale Bereich federelastisch ist. Das magnetische Element kann als zylindrischer Hohlkörper ausgeführt werden, der auf das Führungselement aufbringbar ist, und Detektionsmoleküle auf seiner Oberfläche trägt.

Der Erfindung liegt die Aufgabe zugrunde, eine Detektionsvorrichtung der eingangs genannten Art anzugeben, mittels der die Diagnose unterschiedlicher Krankheiten mit verringertem Aufwand und verbesserter Diagnosepräzision erfolgen kann.

Um die der Erfindung zugrunde liegende Aufgabe zu lösen, wird gemäß dem Gegenstand des Anspruchs 1 eine Detektionsvorrichtung zur in vivo und/oder in vitro Anreicherung von Probenmaterial bereit gestellt, welche wenigstens ein Führungselement zur Führung zumindest eines Funktionselements und zumindest zwei an dem Führungselement angeordnete Funktionselemente aufweist, an denen jeweils eine mit Detektionsrezeptoren bestückte Funktionsfläche ausgebildet ist, wobei die Funktionselemente voneinander lösbar ausgebildet und/oder einzeln von dem Führungselement lösbar sind, wobei zumindest ein als ringförmiges Segment ausgebildetes Funktionselement auf das Führungselement aufgeschraubt ist und/oder dass zumindest ein als flexibler Draht oder Faden ausgebildetes Funktionselement an dem distalen Ende des Führungselements stoffschlüssig befestigt ist.

Durch die voneinander lösbare Ausgestaltung der Funktionselemente und/oder durch die einzeln von dem Führungselement lösbare Anordnung der Funktionselemente können diese unterschiedlichen Diagnoseverfahren unabhängig voneinander zugeführt werden. Die Diagnostik unterschiedlicher Krankheiten kann hierdurch zielgerichteter und damit unter verringertem Aufwand durchgeführt werden.

Die Funktionsfläche eines Funktionselementes kann mit chemisch identischen oder chemisch verschiedenen Detektionsrezeptoren bestückt sein. Somit können bei einer Anwendung nach Bedarf auch verschiedene Liganden an einer Funktionsfläche angereichert werden. In bevorzugter Weise ist die Funktionsfläche eines Funktionselements jedoch mit Detektionsrezeptoren oder einer Kombination von Detektionsrezeptoren bestückt, die sich von den Detektionsrezeptoren oder der Kombination von Detektionsrezeptoren auf der Funktionsfläche des jeweils anderen Funktionselements unterscheiden.

Im Sinne dieser Erfindung werden alle Strukturen, insbesondere Rezeptoren oder Liganden, die geeignet sind, Zielmoleküle und Zielzellen einzufangen, als Detektionsrezeptoren bezeichnet. Ferner werden alle Zielmoleküle und Zielzellen, die an den Detektionsrezeptoren andocken können, vereinfachend als Liganden bezeichnet. Der Begriff Probenflüssigkeit bezeichnet eine in flüssiger Form vorliegende Probe.

Im folgenden soll sich die Bezeichnung proximal auf eine Richtung, ein Ende beziehungsweise einen Endabschnitt beziehen, die oder der einem potentiellen Bediener der Detektionsvorrichtung zugeordnet ist. Demgegenüber soll sich die Bezeichnung distal auf eine Richtung, ein Ende beziehungsweise einen Endabschnitt beziehen, die oder der einem potentiellen Patienten oder eines zu untersuchenden Probenmaterials zugeordnet ist.

Bevorzugte Weiterbildungen der Erfindung sind Gegenstände der Unteransprüche.

In einer vorteilhaften Ausführung der Erfindung erfüllt das Führungselement vorzugsweise wenigstens eine der folgenden Anforderungen:
- Das Führungselement ist zumindest abschnittsweise als Draht ausgebildet.
- Das Führungselement ist zumindest abschnittsweise federelastisch ausgebildet.
- Das Führungselement ist zumindest abschnittsweise als flexibler medizinischer Führungsdraht ausgebildet.
- Das Führungselement ist zumindest abschnittsweise als Faden ausgebildet.
- Das Führungselement ist zumindest abschnittsweise als flexibler Kunststofffaden ausgebildet.
- Das Führungselement ist zumindest abschnittsweise als Katheter ausgebildet.
- Das Führungselement weist einen Aufnahmeabschnitt zur Aufnahme zumindest eines Funktionselements auf.
- Das Führungselement weist ein distales Ende sowie ein proximales Ende auf, wobei zwischen dem distalen und dem proximalen Ende ein Aufnahmeabschnitt zur Aufnahme zumindest eines Funktionselements ausgebildet ist.
- Das Führungselement ist zumindest abschnittsweise schraubenförmig ausgebildet.
- Das Führungselement weist an seinem Außenumfang zumindest abschnittsweise ein Außengewinde auf.
- Das Führungselement weist ein distales Ende sowie ein proximales Ende auf, wobei das distale Ende in ein Blutgefäß einführbar ist.
- Das Führungselement weist ein distales sowie ein proximales Ende auf, wobei das distale Ende verdickt ausgebildet ist.
- Das Führungselement weist ein distales Ende sowie ein proximales Ende auf, wobei das proximale Ende mit einem Stabilisierungselement verbunden ist.
- Das Führungselement ist in ein Innengewinde des Stabilisierungselements eingeschraubt.
- Das Führungselement ist aus einem metallischen und/oder nicht-metallischen Material gefertigt.
- Das Führungselement weist an seinem distalen Abschnitt eine mit Detektionsrezeptoren bestückte Funktionsfläche auf.

Gemäß einer weiteren vorteilhaften Ausführung der Erfindung ist das Führungselement mit einem Stabilisierungselement zur Stabilisierung des wenigstens einen Führungselements verbunden, welches vorzugsweise wenigstens eine der folgenden Anforderungen erfüllt:
- Das Stabilisierungselement ist dazu ausgebildet, das Führungselement zumindest abschnittsweise zu stabilisieren.
- Das Stabilisierungselement ist aus Kunststoff oder Metall gefertigt.
- Das Stabilisierungselement ist mit einem proximalen Ende oder einem proximalen Endabschnitt des Führungselements verbunden.
- Das Stabilisierungselement ist lösbar mit dem Führungselement verbunden.
- Das Stabilisierungselement ist form-, kraft- und/oder stoffschlüssig mit dem Führungselement verbunden.
- Das Stabilisierungselement ist mit dem Führungselement verklebt oder verschweißt.
- Das Stabilisierungselement ist zumindest abschnittsweise zylindrisch ausgebildet.
- Das Stabilisierungselement ist zumindest abschnittsweise als Hülse ausgebildet.
- Das Stabilisierungselement weist zumindest abschnittsweise ein Innengewinde auf.
- Das Stabilisierungselement ist zumindest abschnittsweise auf einen proximalen Abschnitt des Führungselementes aufgeschoben, aufgesteckt oder aufgeschraubt.
- Das Stabilisierungselement ist zumindest abschnittsweise auf einen distalen Abschnitt des Führungselementes aufgeschoben, aufgesteckt oder aufgeschraubt.
- Das Stabilisierungselement führt das Führungselement zumindest abschnittsweise.
- Das Stabilisierungselement führt das Führungselement beweglich.
- Das Stabilisierungselement ist als Abdeckvorrichtung für zumindest ein Funktionselement ausgebildet.
- Das Stabilisierungselement weist eine Aufnahme auf, in der die zumindest zwei Funktionselemente angeordnet sind.
- Das Stabilisierungselement ist in ein Blutgefäß einführbar.
- Das Stabilisierungselement weist eine Aufnahme auf, in der die zumindest zwei Funktionselemente herausführbar angeordnet sind.
- Das Stabilisierungselement weist ein distales und ein proximales Ende auf, wobei an dem distalen Ende ein Gewinde, insbesondere ein Luer-Lock-Gewinde zum Anschließen von Verweilkanülen, ausgebildet ist.
- Das Stabilisierungselement weist eine Druck-Zug-Vorrichtung auf, mittels der die zumindest zwei Funktionselemente aus der Hülse heraus- und wieder in diese hineinführbar sind.
- Das Stabilisierungselement weist zumindest einen Abschnitt auf, dessen Aussendurchmesser dem Aussendurchmesser des distalen Abschnitts des Führungselements entspricht oder im Wesentlichen entspricht.
- Das Stabilisierungselement ist mit dem Führungselement in einem Herstellverfahren gefertigt.
- Das Stabilisierungselement weist ein abgerundetes Ende als Verletzungsschutz auf.
- Das Stabilisierungselement ist zur Stabilisierung der Verbindungsstelle zwischen zumindest einem der Funktionselemente sowie dem Führungselement ausgebildet.

Gemäß einer noch weiteren vorteilhaften Ausgestaltung der Erfindung ist ferner eine Abdeckvorrichtung vorgesehen, die wenigstens eine der folgenden Anforderungen erfüllt:
- Die Abdeckvorrichtung ist zur Abdeckung zumindest eines der Funktionselemente ausgebildet.
- Die Abdeckvorrichtung ist Bestandteil des Stabilisierungselements.
- Die Abdeckvorrichtung ist als von dem Stabilisierungselement diskrete Komponente ausgebildet.
- Die Abdeckvorrichtung ist dazu ausgebildet, das Führungselement zumindest abschnittsweise zu stabilisieren.
- Die Abdeckvorrichtung ist aus Kunststoff oder Metall gefertigt.
- Die Abdeckvorrichtung ist zumindest abschnittsweise zylindrisch ausgebildet.
- Die Abdeckvorrichtung weist eine longitudinale Bohrung auf, zur Aufnahme zumindest eines Funktionselements.
- Die Abdeckvorrichtung ist zumindest abschnittsweise als Hülse ausgebildet.
- Die Abdeckvorrichtung ist zumindest abschnittsweise auf einen proximalen Abschnitt des Führungselementes aufgeschoben, aufgesteckt oder aufgeschraubt.
- Die Abdeckvorrichtung ist in ein Blutgefäß einführbar.
- Die Abdeckvorrichtung weist eine Aufnahme auf, in der die zumindest zwei Funktionselemente herausführbar angeordnet sind.
- Die Abdeckvorrichtung ist entlang einer Längsorientierung relativ zu den Funktionselementen und/oder relativ zum Stabilisierungselement beweglich angeordnet.
- Die Abdeckvorrichtung weist ein distales und ein proximales Ende auf, wobei an dem distalen Ende ein Gewinde, insbesondere ein Luer-Lock-Gewinde zum Anschließen von Verweilkanülen, ausgebildet ist.
- Die Abdeckvorrichtung weist Homopolymere, Copolymere, Biopolymere, chemisch modifizierte Polymere und/oder synthetische Polymere auf.
- Die Abdeckvorrichtung weist ein abgerundetes Ende als Verletzungsschutz auf.
- Die Abdeckvorrichtung ist zur Abdeckung der Verbindungsstelle zwischen zumindest einem der Funktionselemente sowie dem Führungselement ausgebildet.

Gemäß einer weiterhin vorteilhaften Ausgestaltung der Erfindung erfüllt zumindest eines der Funktionselemente wenigstens eine der folgenden Anforderungen erfüllt:
- Das Funktionselement ist zumindest teilweise aus Metall, vorzugsweise Edelstahl, medizinischem Edelstahl oder Titan; aus Glas, vorzugsweise Glasfaser; aus Kunststoff, vorzugsweise einem geschäumten Kunststoff, einem Polymer, bevorzugt Polyethylen, Polypropylen, Polyurethan, Polytetrafluorethylen, einem auf organischen Polymeren basierenden Kunststoff oder einer Kombination dieser Materialien gefertigt.
- Das Funktionselement ist als flexibler Faden ausgebildet, der bevorzugt einen geringere Dicke und/oder eine größere Flexibilität als das Führungselement aufweist.
- Das Funktionselement ist an dem distalen Ende des Führungselements, insbesondere an einer Querschnittsfläche des distalen Endes, befestigt.
- Das Funktionselement ist an dem Außenumfang des Führungselements befestigt, wobei die Länge des Funktionselements bevorzugt größer ist als der Abstand der Verbindungsstelle am Außenumfang des Führungselements vom distalen Ende des Führungselements.
- Das Funktionselement ist an seinem distalen Ende mit wenigstens einem weiteren Funktionselement, vorzugsweise mit wenigstens zwei weiteren Funktionselementen verbunden, vorzugsweise stoffschlüssig verbunden.
- Das Funktionselement ist als Draht, insbesondere als flexibler Draht ausgebildet, der bevorzugt einen geringere Dicke und/oder eine größere Flexibilität als das Führungselement aufweist.
- Das Funktionselement ist an dem distalen Ende des Führungselements, insbesondere an einer Querschnittsfläche des distalen Endes, befestigt, insbesondere stoffschlüssig befestigt, insbesondere an der Querschnittsfläche des distalen Endes angeschweißt.
- Das Funktionselement ist an seinem distalen Ende mit wenigstens einem weiteren Funktionselement, vorzugsweise mit wenigstens zwei weiteren Funktionselementen verbunden, insbesondere stoffschlüssig verbunden, insbesondere verschweißt.
- Das Funktionselement ist zerstörungsfrei von dem zumindest einen anderen Funktionselement lösbar und/oder zerstörungsfrei von dem Führungselement abnehmbar.
- Das Funktionselement ist vom Führungselement abschneidbar oder abreißbar ist.
- Das Funktionselement ist durch ein ringförmiges Segment gebildet.
- Das Funktionselement weist eine Kugelform oder eine Knochenform auf.
- Das Funktionselement umfasst an dessen Innenumfangsfläche ein Innengewinde.
- Das Funktionselement umschließt zumindest einen Abschnitt des Führungselements.
- Das Funktionselement ist auf das Führungselement aufgeschoben, aufgesteckt oder aufgeschraubt.
- Das Funktionselement ist zwischen einem distalen Ende sowie einem proximalen Ende des Führungselements an diesem angeordnet.
- Das Funktionselement weist Detektionsrezeptoren auf, die sich von den Detektionsrezeptoren des jeweils anderen Funktionselements unterscheiden.
- Das Funktionselement weist eine Kombination von Detektionsrezeptoren auf, die sich von der Kombination von Detektionsrezeptoren des jeweils anderen Funktionselements unterscheidet.
- Das Funktionselement weist zumindest einen Abschnitt mit einem Aussendurchmesser auf, der dem Aussendurchmesser des distalen Abschnitts des Führungselements entspricht oder im Wesentlichen entspricht.
- Das Funktionselement weist zumindest einen Abschnitt mit einem Aussendurchmesser auf, der größer oder kleiner ist als der distale Abschnitt des Führungselements, insbesondere 0,01 mm bis 0,1 mm kleiner ist als der distale Abschnitt des Führungselements.
- Das Funktionselement ist als Stent, Katheter oder Katheterabschnitt ausgebildet.
- Das Funktionselement ist als Mandrain ausgebildet.
- Das Funktionselement ist mit Erhöhungen, Vertiefungen und/oder Verzweigungen ausgebildet.
- Das Funktionselement umfasst zumindest teilweise eine spiralförmige, schraubenförmige, schneckenförmige, wellenförmige, helikale, filamentöse, bürstenförmige, kammförmige, netzförmige, poröse, schwammförmige oder dergleichen Struktur.
- Das Funktionselement umfasst eine Beschichtung aus Metall, vorzugsweise aus einem Metall aus der 10. oder 11. Gruppe des Periodensystems der Elemente, bevorzugt aus Nickel, Kupfer, Palladium, Silber, Platin und/oder Gold.

Gemäß einer weiteren Ausgestaltung der Erfindung erfüllt die Funktionsfläche zumindest eines Funktionselements wenigstens eine der folgenden Anforderungen:
- Die Funktionsfläche weist eine dreidimensionale Struktur mit einander zugewandten Funktionsabschnitten auf, die wenigstens einen von einer Probenflüssigkeit durchsetzbaren Zwischenraum bilden, wobei der Zwischenraum vorzugsweise wenigstens abschnittsweise kanalförmig ausgebildet ist, wobei bevorzugt mehrere Zwischenräume vorzugsweise ein verzweigtes Netz von Kanälen bilden.
- Die Funktionsfläche ist im makroskopischen und/oder im mikroskopischen Bereich dreidimensional strukturiert.
- Die Funktionsfläche ist mit Erhöhungen, Vertiefungen und/oder Verzweigungen ausgebildet.
- Die Funktionsfläche umfasst zumindest teilweise eine spiralförmige, schraubenförmige, schneckenförmige, wellenförmige, helikale, filamentöse, bürstenförmige, kammförmige, netzförmige, poröse, schwammförmige oder dergleichen Struktur.

Die Funktionsabschnitte gelten als einander zugewandt, wenn sie einen Winkel von kleiner als 180 Grad einschließen, sich gegenseitig also "sehen" können. Dadurch weist die Detektionsvorrichtung gegenüber einer Detektionsvorrichtung mit einer glatten beziehungsweise zylindrischen Funktionsfläche eine größere Funktionsfläche auf. Ferner kann die Probenflüssigkeit in den Zwischenräumen ideal über die Funktionsfläche geleitet werden und an den Detektionsrezeptoren angereichert werden. Zudem können die an den Detektionsrezeptoren andockenden Liganden in den Zwischenräumen festgehalten und so vor Abrieb besser geschützt werden. Vorzugsweise kann die Funktionsfläche auch ein gewisses Volumen an Probenflüssigkeit speichern.

Es kann sich als hilfreich erweisen, wenn die Funktionsfläche zumindest eines Funktionselements im makroskopischen und/oder mikroskopischen Bereich dreidimensional strukturiert ist. Durch eine im makroskopischen bzw. sichtbaren Bereich dreidimensional strukturierte Funktionsfläche, die bspw. durch die sichtbare Geometrie der Detektionsvorrichtung bestimmt ist, kann die Probenflüssigkeit in vorteilhafter Weise über die Funktionsfläche geführt werden. Durch eine im mikroskopischen Bereich dreidimensional strukturierte Funktionsfläche kann die Strömungsgeschwindigkeit im Bereich der Grenzschicht verringert werden. Die Zwischenräume sind vorzugsweise mindestens derart dimensioniert, dass spezifische Liganden an den Detektionsrezeptoren andocken können. Vorzugsweise sind die Zwischenräume derart dimensioniert, dass in etwa die Anzahl an spezifischen Liganden aufgenommen und angeordnet werden können, die der Anzahl der Detektionsrezeptoren an den einander zugewandten Funktionsabschnitten entspricht. Dadurch wird die Funktionsfläche in idealer Weise genutzt. Ferner können die Liganden vor Abrieb noch besser geschützt werden.

In einer vorteilhaften Ausführung der Erfindung ist der Zwischenraum wenigstens abschnittsweise kanalförmig ausgebildet, wobei mehrere Zwischenräume vorzugsweise ein verzweigtes Netz von Kanälen bilden. Der Kanal kann sich über die gesamte Länge der Funktionsfläche erstrecken. Dadurch kann Probenflüssigkeit ideal zu den Detektionsrezeptoren geleitet werden. Durch die Auslegung der Größe der Zwischenräume kann eine Fließgeschwindigkeit und Fließrichtung der Probenflüssigkeit beeinflusst werden. Vorzugsweise erstreckt sich der Kanal zumindest abschnittsweise in Längsrichtung der Detektionsvorrichtung, so dass durch den Kanal eine natürliche Fließrichtung der Probenflüssigkeit möglichst wenig beeinträchtigt wird.

Es kann von Vorteil sein, wenn die Funktionsfläche zumindest eines Funktionselements mit Erhöhungen, Vertiefungen und/oder Verzweigungen ausgebildet ist, und/oder zumindest teilweise eine spiralförmige, schraubenförmige, schneckenförmige, wellenförmige, helikale, filamentöse, bürstenförmige, kammförmige, netzförmige, poröse, schwammförmige oder dergleichen Struktur umfasst. Derartige Formen besitzen große Oberflächen und sind für die Anwendung in der erfindungsgemäßen Detektionsvorrichtung gut geeignet. Spiralförmige, schraubenförmige, schneckenförmige, wellenförmige, helikale Strukturen weisen i.d.R. einen geringen Reibungswiderstand auf. Dadurch wird die Strömung der Probenflüssigkeit, bspw. Blut in einem Blutgefäß, wenig beeinträchtigt, selbst wenn Teile der Funktionsfläche an den Gefäßwänden anliegen. Filamentöse, bürstenförmige, kammförmige, netzförmige, poröse und schwammförmige Strukturen verlangsamen die natürliche Strömung der Probenflüssigkeit und eignen sich hervorragend als Flüssigkeitsspeicher, wodurch die Anreicherung der Liganden an den Detektionsrezeptoren zusätzlich begünstigt wird.

Es kann sich als vorteilhaft erweisen, wenn die Detektionsrezeptoren Antikörper, Antikörperfragmente, Aminosäuren-Strukturen, Nukleinsäure-Strukturen, anorganische Materialen und/oder synthetische Strukturen mit spezifischer Affinität zu Zelloberflächen umfassen, vorzugsweise monoklonale Antikörper murinen Ursprungs, chimäre Antikörper oder humanisierte Antikörper, bevorzugt HLA-G und/oder EpCAM Antikörper.

Es kann auch praktisch sein, wenn die Funktionsfläche zumindest eines Funktionselements gesättigte Atomgruppen und kovalent gebundene Liganden und Rezeptoren umfasst, um unerwünschte Wechselwirkungen mit Blutbestandteilen und die Anbindung von unspezifischen Zellen und Molekülen zu verhindern.

Die Handhabung der Detektionsvorrichtung kann ferner dadurch erleichtert werden, dass das Führungselement, die zumindest zwei Funktionselemente, das Stabilisierungselement und/oder die Abdeckvorrichtung zu einem Stilett zusammengefügt sind.

Es kann aber auch praktisch sein, wenn dass das Führungselement, das Stabilisierungselement und/oder die Abdeckvorrichtung eine Markierung zur Applikationskontrolle aufweist. Dadurch kann die Detektionsvorrichtung im Einsatz besser kontrolliert werden.

In einer anderen vorteilhaften Ausführung der Erfindung weist zumindest eines der Funktionselemente ein biokompatibles Polymer auf, das vorzugsweise wenigstens eine der folgenden Anforderungen erfüllt:
- Das biokompatible Polymer ist als zusammenhängende Polymerschicht ausgebildet. Dadurch kann die gesamte Oberfläche des Funktionselements durch die Polymerschicht abgedeckt oder abgeschirmt werden. Die Dicke der Polymerschicht liegt vorzugsweise im Bereich von 0,1 bis 10 µm, bevorzugt im Bereich von 0,5 bis 5 µm, besonders bevorzugt im Bereich von 1 bis 2 µm.
- Das biokompatible Polymer weist eine dreidimensionale, vorzugsweise filamentöse und/oder poröse Struktur auf. Diese Struktur bildet viele Zwischenräume, welche durch die an den Detektionsrezeptoren andockenden Liganden im Wesentlichen gefüllt werden, so dass die Liganden vor Abrieb noch besser geschützt sind. Das biokompatible Polymer kann im makroskopischen und/oder im mikroskopischen Bereich dreidimensional strukturiert sein.
- Das biokompatible Polymer weist eine dreidimensionale, vorzugsweise filamentöse, und/oder poröse Oberfläche auf. Die Oberfläche des biokompatiblen Polymers kann im makroskopischen und/oder mikroskopischen Bereich dreidimensional strukturiert sein.
- Das biokompatible Polymer weist eine kohlenstoffhaltige, verzweigte Molekülstruktur auf. Diese Molekülstruktur eignet sich hervorragend für die Anbindung der Detektionsrezeptoren und die Anreicherung von Liganden an den Detektionsrezeptoren. Dabei bildet diese Molekülstruktur eine filamentöse Funktionsfläche im Sinne der Erfindung, die im mikroskopischen Bereich dreidimensional strukturiert ist. Die verzweigten Molekülstrukturen umfassen zahlreiche Zwischenräume, die von gegenüberstehenden und mit Detektionsrezeptoren bestückten Funktionsabschnitten in Form von Polymermolekülen gebildet sind. Diese Zwischenräume sind von einer Probenflüssigkeit durchsetzbar und bilden einen Flüssigkeitsspeicher, wodurch die Anreicherung der Liganden an den Detektionsrezeptoren besonders begünstigt wird. Im Bereich der Grenzschicht an einer mit dieser Molekülstruktur besetzten Oberfläche wird die Umströmung von einer Probenflüssigkeit erheblich verlangsamt. Dadurch wird die Anreicherung der Liganden zusätzlich begünstigt.
- Das biokompatible Polymer ist vorzugsweise über funktionelle Gruppen mit dem Funktionselement wirkverbunden, bevorzugt durch chemische Bindung, besonders bevorzugt durch kovalente Bindung.
- Das biokompatible Polymer umfasst funktionelle Gruppen, vorzugsweise Carboxyl-Gruppen, wobei die funktionellen Gruppen vorzugsweise durch chemische Aktivierung eine unausgeglichene Molekülladung aufweisen, wobei die funktionellen Gruppen bevorzugt auf die Detektionsrezeptoren abgestimmt sind.
- Das biokompatible Polymer weist hydrophile Eigenschaften auf und ist vorzugsweise ein Hydrogel.
- Das biokompatible Polymer umfasst chemisch und/oder enzymatisch spaltbare Gruppen, welche die Ablösung der Liganden erleichtert. Die chemisch und/oder enzymatisch spaltbaren Gruppen sind vorzugsweise die funktionellen Gruppen, an welchen die Detektionsrezeptoren gebunden sind.
- Das biokompatible Polymer umfasst gesättigte Atomgruppen und kovalent gebundene Detektionsrezeptoren, um unerwünschte Wechselwirkungen mit Blutbestandteilen und die Anbindung von unspezifischen Zellen und Molekülen zu verhindern.
- Das biokompatible Polymer ist in einem Hohlraum des Funktionselements angeordnet. Dadurch kann ein Hohlraum des Funktionselements zur Anreicherung von Liganden genutzt werden. Dort sind die Liganden vor Abrieb optimal geschützt.
- Das biokompatible Polymer ist vernetzt.
- Das biokompatible Polymer umfasst bzw. bildet die Funktionsfläche. Die Funktionsfläche befindet sich vorzugsweise an der Oberfläche des biokompatiblen Polymers. Das biokompatible Polymer kann direkt mit den Detektionsrezeptoren bestückt sein.

In noch einer anderen vorteilhaften Ausführung der Erfindung ist die Funktionsfläche mit einer Schutzschicht überzogen, wobei die Schutzschicht vorzugsweise wenigstens eine der folgenden Anforderungen erfüllt:
- Die Schutzschicht ist in Flüssigkeiten, insbesondere in Körperflüssigkeiten, vorzugsweise in Blut, löslich. Dadurch kann die Funktionsfläche automatisch freigelegt werden, sobald die Schutzschicht in Kontakt mit der Probenflüssigkeit gelangt.
- Die Schutzschicht ist biokompatibel. Dadurch werden Abwehrreaktionen des Körpers bei der in vivo Anwendung der Detektionsvorrichtung weitgehend unterbunden.
- Die Schutzschicht ist organisch kristallin und umfasst wenigstens einen der folgenden Bestandteile: Alginate, vorzugsweise hochreine Alginate, Polyethylenglykole, zyklische und nicht zyklische Oligosaccharide, Polysaccharide, antioxidative Aminosäuren, Proteine oder Vitamine. Derartige Bestandteile sind biokompatibel und leicht löslich.

Es kann nützlich sein, wenn die Detektionsrezeptoren vorzugsweise über Linker oder organische funktionelle Gruppen mit der Funktionsfläche, vorzugsweise mit dem Träger und/oder dem biokompatiblen Polymer, wirkverbunden sind, vorzugsweise durch chemische Bindung, bevorzugt durch kovalente Bindung. Dadurch kann eine unspezifische Adsorption an der Funktionsfläche weitgehend verhindert werden.

Ein weiterer unabhängiger Aspekt der Erfindung betrifft eine Detektionsvorrichtung, vorzugsweise nach wenigstens einer der vorangegangenen Ausführungen, hergestellt durch:
- Bereitstellung einer Detektionsvorrichtung mit wenigstens einem Führungselement zur Führung zumindest eines Funktionselements und zumindest zwei Funktionselementen,
- chemische Aktivierung der Funktionselemente durch chemische, vorzugsweise kovalente Bindung der Detektionsrezeptoren direkt oder über ein biokompatibles Polymer an funktionelle Gruppen der Funktionselemente, vorzugsweise an organische funktionelle Gruppen der Funktionselemente, bevorzugt über schwefel- und/oder stickstoffhaltige Verbindungen.

Die Detektionsvorrichtung kann jedes der zuvor genannten Merkmale aufweisen.

Noch ein weiterer unabhängiger Aspekt der Erfindung betrifft die Verwendung einer Detektionsvorrichtung nach einer der vorangegangenen Ausführungen zur Anreicherung von Proben, vorzugsweise aus dem Blutgefäßsystem, aus ableitenden Drüsengängen der Bauchspeicheldrüse, aus Tränendrüsen, aus Ohrspeicheldrüsen, aus ableitenden Drüsengängen der mukösen Drüsen, aus gemischten Drüsen, aus Haut- und Hautanhangsdrüsen, aus Milchdrüsen, aus Spinalkanälen, Hirnkammersystemen, aus Periduralräumen, aus Gallenblasen und ihren ableitenden anatomischen Strukturen, aus ableitenden Harnwegen oder lymphatischen Systemen, aus Körperhöhlen des Bauches, des Brustkorbes, der Gebärmutter, des Urogenitalapparates, eines Gelenkes oder des Gastrointestinaltraktes.

Bei der Verwendung einer Detektionsvorrichtung nach einer der vorangegangenen Ausführungen kann die Detektionsvorrichtung unabhängig vom Verwendungszweck über einen Gefäßzugang in ein Gefäßsystem eingebracht werden.

Es versteht sich, dass eine Detektionsvorrichtung nach einer der vorangegangenen Ausführungen für endoskopische Applikationen verwendet werden kann.

Insbesondere betrifft ein Aspekt der Erfindung die Verwendung einer Detektionsvorrichtung nach einer der vorangegangenen Ausführungen zur invasiven Anreicherung von Probenmaterial, zur Elimination von Arzneimittel, zur Elimination von radioaktiven Tracern, zur Elimination von Magnetobeads, zur Gewinnung von Tumor- oder Biomarkern und/oder zur Elimination von Toxinen, oder zur Anreicherung von Zellen, umfassend embryonale Trophoblasten, disseminierten Tumorzellen, insbesondere von hämatogen metastasierenden Tumoren. Dadurch lassen sich die genannten Vorteile erzielen.

Weiterhin kann in vorteilhafter Weise eine Detektionsvorrichtung nach einer der vorangegangenen Ausführungen zur invasiven Anreicherung von Probenmaterial, zur Elimination von Arzneimittel, zur Elimination von radioaktiven Tracern, zur Elimination von Magnetobeads, zur Gewinnung von Tumor- oder Biomarkern und/oder zur Elimination von Toxinen, oder zur Anreicherung von Zellen, umfassend embryonale Trophoblasten, disseminierten Tumorzellen, insbesondere von hämatogen metastasierenden Tumoren, verwendet werden.

Die Erfindung betrifft aber auch die Verwendung einer Detektionsvorrichtung nach einer der vorangegangenen Ausführungen zur Anreicherung von Proben zur Diagnose, insbesondere pränatalen Diagnose, Krebsdiagnose und der Therapie-Verlaufskontrolle sowie zur Diagnose einer Erkrankung die ausgewählt ist aus der Gruppe bestehend aus Erberkrankungen, proliferativen Erkrankungen, inflammatorischen Erkrankungen, Autoimmunerkrankungen, Infektionserkrankungen, hormonellen Erkrankungen, Erkrankungen des Blutes und der blutbildenden Organe, Erkrankungen des Verdauungstraktes, der Leber, der Galle, der Bauchspeicheldrüse, Erkrankungen des Urogenitaltraktes und der Niere, Erkrankungen des Herzens, krankhafte Veränderungen des Blutgefäßsystems und des Lymphsystems, Erkrankungen der Lunge, Erkrankungen des zentralen oder peripheren Nervensystems sowie der elektrischen Reizweiterleitung und/oder neurodegenerative Erkrankungen.

Dabei kann die pränatale Diagnose die Diagnose von Genmutationen, Chromosomenmutationen und Chromosomenaberrationen aus der Gruppe einer Deletion, einer Inversion, einer Duplikation, einer Translokation von Ringchromosomen, einer Störung der Gen-Transkription, der Gen-Translation, der mRNA-Stabilität, einer Splice-Varianten, einer Störung des mRNA Transportes ins Cytoplasma, der Protein-Biosynthese und/oder eines epigenetischen Faktors, umfassen.

Vorliegend kann die Krebsdiagnose die Primärdiagnostik, die Diagnostik zur Tumorausbreitung und/oder das Tumor Grading umfassen. Ferner kann die Therapie-Verlaufskontrolle die Überwachung einer Tumorbehandlung, die Überwachung einer autologen, syngenen, allogenen, xenogenen oder alloplastischen Transplantation, die Überwachung einer entzündlichen Erkrankung, die Überwachung einer Infektionserkrankung, die Überwachung einer hormonellen Erkrankung, die Überwachung einer psychiatrischen Erkrankung und/oder die Überwachung einer neuro-degenerativen Erkrankung umfassen.

Erberkrankungen, zu deren Diagnose eine Detektionsvorrichtung nach einer der vorangegangenen Ausführungen verwendet werden kann, können ausgewählt sein aus der Gruppe umfassend autosomal rezessive, autosomal dominante, gonosomale, mitochondriale und/oder extrachromosomale Erberkrankungen und/oder Erkrankungen, die auf eine genetische Disposition zurück geführt werden.

Hingegen kann es sich bei proliferativen Erkrankungen, die unter Verwendung einer Detektionsvorrichtung nach einer der vorangegangenen Ausführungen diagnostiziert werden, um Tumore, Präcancerosen, Dysplasien, neuroendokrine Tumore, Endometriosen und/oder Metaplasien handeln.

Weiterhin erstreckt sich die Erfindung aber auch auf die Verwendung einer Detektionsvorrichtung nach einer der vorangegangenen Ausführungen zur Anreicherung von Proben zur Diagnose einer Autoimmunerkrankung, wobei die Autoimmunerkrankung ausgewählt ist aus der Gruppe umfassend rheumatoide Arthritis, inflammatorische Darmerkrankung, Osteoarthritis, neuropathische Schmerzen, Alopecia areata, Psoriasis, psoriathrische Arthritis, akute Pankreatitis, Allograftabstoßung, Allergien, allergische Entzündungen der Lunge, Multiple Sklerose, Alzheimer, Morbus Crohn und/oder systemischer Lupus erythematous.

Ebenso kann eine Detektionsvorrichtung nach einer der vorangegangenen Ausführungen zur Anreicherung von Proben zur Diagnose einer Infektionserkrankung verwendet werden, wobei die Infektionserkrankung ausgewählt ist aus der Gruppe umfassend parasitäre Erkrankungen, bakterielle Erkrankungen und/oder einer virale Erkrankungen.

Hormonelle Erkrankungen, die unter Verwendung einer Detektionsvorrichtung nach einer der vorangegangenen Ausführungen diagnostiziert werden können, können ausgewählt sein aus der Gruppe umfassend Erkrankung des Zuckerstoffwechsels, des Fettstoffwechsels, des Proteinstoffwechsels, der sexuellen Entwicklung und Fortpflanzung, des Wasser-Salz-Haushaltes, des Wachstums und/oder der Zellbildung.

Noch ein weiterer unabhängiger Aspekt der Erfindung betrifft ein Verfahren zur Anreicherung von Probenmaterial unter Benutzung einer Detektionsvorrichtung nach wenigstens einer der vorangegangenen Ausführungen, wobei das Verfahren die folgenden Schritte umfasst:
- Bereitstellen einer Detektionsvorrichtung nach wenigstens einem der vorangegangenen Ansprüche,
- Einführen der Detektionsvorrichtung in einen lebenden Organismus,
- Beaufschlagen der Funktionsflächen der zumindest zwei Funktionselemente mit einer Probenflüssigkeit des lebenden Organismus,
- Anreicherung von Probenmaterial, vorzugsweise Zellen, DNA, RNA, Proteine, Peptide, synthetische Moleküle, an den Detektionsrezeptoren,
- Entnahme der Detektionsvorrichtung aus dem lebenden Organismus.

Nach den voranstehenden Verfahrensschritten können die Funktionselemente in vorteilhafter Weise voneinander gelöst und/oder einzeln von dem Führungselement gelöst werden und anschließend unabhängig voneinander für ein jeweils geeignetes Diagnoseverfahren bereitgestellt werden.

Die Detektionsvorrichtung kann jedes der zuvor genannten Merkmale aufweisen.

Bevorzugte Weiterbildungen der Erfindung ergeben sich durch Kombinationen der Unteransprüche oder der darin genannten Teilmerkmale.

### Kurze Beschreibung der Figuren

- Figur 1: ist eine schematische Darstellung einer erfindungsgemäßen Detektionsvorrichtung gemäß einem ersten Ausführungsbeispiel.
- Figur 2: ist eine schematische Querschnittsdarstellung eines Funktionselements gemäß einem ersten Ausführungsbeispiel.
- Figur 3: ist eine schematische Darstellung einer erfindungsgemäßen Detektionsvorrichtung gemäß einem zweiten Ausführungsbeispiel.
- Figur 4: ist eine schematische Darstellung eines Funktionselements gemäß einem zweiten Ausführungsbeispiel in geschnittener Längsansicht.
- Figur 5a: ist eine schematische Darstellung einer erfindungsgemäßen Detektionsvorrichtung gemäß einem dritten Ausführungsbeispiel.
- Figur 5b: ist eine schematische Darstellung einer erfindungsgemäßen Detektionsvorrichtung gemäß einem vierten Ausführungsbeispiel.
- Figur 6: zeigt einen beispielhaften Schichtaufbau eines Trägers.

### Detaillierte Beschreibung der bevorzugten Ausführungsbeispiele

Die bevorzugten Ausführungsbeispiele der Erfindung werden nachstehend mit Bezug auf die beigefügten Zeichnungen beschrieben.

### Erstes Ausführungsbeispiel

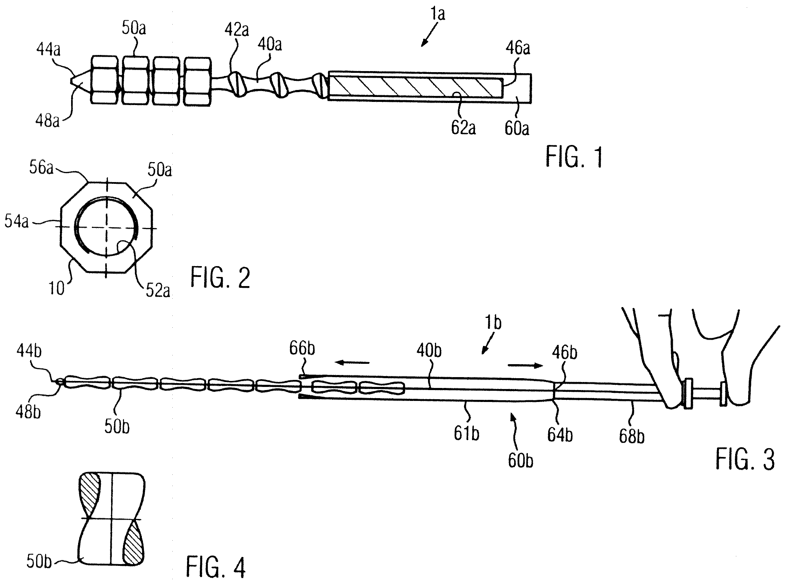

Figur 1 ist eine schematische Darstellung eines Detektionskatheters 1a gemäß einem ersten Ausführungsbeispiel. Die erfindungsgemäße Detektionsvorrichtung 1a nach dem ersten Ausführungsbeispiel umfasst einen flexiblen medizinischen Führungsdraht 40a, der an seinem Außenumfang eine Gewindestruktur, insbesondere ein Außengewinde 42a, aufweist. Der Führungsdraht 40a weist ferner ein distales Ende 44a sowie ein proximales Ende 46a auf. An dem distalen Ende 44a des Führungsdrahtes 40a ist eine kegelförmige Begrenzung 48a ausgebildet.

Weiterhin umfasst die Detektionsvorrichtung 1a ein Stabilisierungselement 60a, welches zur Stabilisierung und Fixierung des Führungsdrahtes 40a angeordnet ist. Das Stabilisierungselement kann als zylinderförmige Hülse 60a ausgeführt sein, an dessen Innenumfangsfläche zumindest abschnittsweise ein Innengewinde 62a ausgebildet ist. Der Führungsdraht 40a kann über sein distales Ende 46a in die zylinderförmige Hülse 60a eingeschraubt werden.

Die Detektionsvorrichtung 1a umfasst ferner wenigstens zwei Funktionselemente 50a, die auf den Führungsdraht 40a aufgeschraubt sind. Vorzugsweise sind mehr als zwei Funktionselemente 50a auf den Führungsdraht 40a aufgeschraubt. Bei den Funktionselementen handelt es sich um prismatische oder zylinderförmige Ringsegmente 50a, an deren Innenumfangsfläche ein Innengewinde 52a ausgebildet ist, wie in Fig. 2 dargestellt. Die Außenumfangsfläche 54a eines Funktionselements 50a kann mit acht Flächenabschnitten sowie die Flächenabschnitte begrenzenden Kanten 56a versehen sein, sodass das jeweilige Funktionselement 50a nach Art einer Schraubenmutter gehandhabt und dadurch mit nur geringem Aufwand auf den Führungsdraht 40a aufgeschraubt werden kann.

Zum Aufschrauben der Funktionselemente 50a auf den Führungsdraht 40a muss das Stabilisierungselement 60a von dem Führungsdraht 40a entfernt sein. Die Funktionselemente 50a können dementsprechend vom proximalen Ende 46a des Führungsdrahtes auf diesen aufgeschraubt werden. Das zuerst aufgeschraubte Funktionselement 50a kommt an der kegelförmigen Begrenzung 48a am distalen Ende 44a des Führungsdrahtes zur Anlage. Jedes weitere Funktionselement 50a, welches auf den Führungsdraht 40a aufgeschraubt wird, kann an dem jeweils zuvor aufgeschraubten Funktionselement 50a zur Anlage kommen. Nach Anordnung der jeweils benötigten Anzahl von Funktionselementes 50a kann der Führungsdraht über sein proximales Ende 46a in das als zylinderförmige Hülse ausgebildete Stabilisierungselement eingeschraubt werden.

Um nach der in vivo und/oder in vitro Anreicherung von Probenmaterial mittels der Detektionsvorrichtung 1a die einzelnen Funktionselemente 50a für nachgeordnete Diagnoseverfahren zur Verfügung zu stellen, wird zunächst der Führungsdraht 40a aus dem Stabilisierungselement 60a ausgeschraubt, sodass anschließend die Funktionselemente 50a einzeln von dem Führungsdraht 40a abgeschraubt werden können.

Jedes der Funktionselemente 50a weist eine mit Detektionsrezeptoren 12 bestückte Funktionsfläche 10 auf, wobei die Funktionsfläche 10 mit einer Sorte von Detektionsrezeptoren 12 oder mit einer Kombination aus Detektionsrezeptoren 12 bestückt sein kann. Dabei kann es von Vorteil sein wenn die Funktionsfläche 10 eines Funktionselements 50a mit Detektionsrezeptoren 12 oder einer Kombination aus Detektionsrezeptoren 12 bestückt ist, die sich von den Detektionsrezeptoren 12 oder der Kombination von Detektionsrezeptoren 12 des zumindest einen anderen Funktionselements 50a unterscheidet. Auf diese Weise kann die Detektionsvorrichtung 1a gezielt für die unterschiedliche Einsatz- beziehungsweise Diagnosezwecke mit Funktionselementen 50a, die unterschiedliche Detektionsrezeptoren 12 beziehungsweise unterschiedliche Kombinationen aus Detektionsrezeptoren 12 aufweisen, konfiguriert werden. Dadurch, dass die Funktionselemente 50a voneinander trennbar angeordnet sind beziehungsweise einzeln von dem Führungsdraht 40a abnehmbar angeordnet sind, können die unterschiedlichen Funktionselemente gezielt unterschiedlichen Diagnoseverfahren zugeführt werden, die der jeweiligen in vivo und/oder in vitro Anreicherung von Probenmaterial nachgeschaltet ist.

### Zweites Ausführungsbeispiel

Figur 3 ist eine schematische Darstellung eines Detektionskatheters 1b gemäß einem zweiten Ausführungsbeispiel. Bei der der erfindungsgemäßen Detektionsvorrichtung 1b nach dem zweiten Ausführungsbeispiel handelt es sich um einen biofunktionalisierten, medizinischen Detektionskatheter für die invasive (in vivo) Anreicherung von seltenen Zellen, Biomolekülen oder Arzneimitteln. Ein derartiger Detektionskatheter beziehungsweise Detektor wird auch als medizinscher Nano-Katheter (MN-K) bzw. medical nano-catheter (MN-C) bezeichnet.

Die erfindungsgemäße Detektionsvorrichtung 1b gemäß dem zweiten Ausführungsbeispiel umfasst ein Führungselement 40b. Das Führungselement 40b kann als flexibler, medizinischer Führungsdraht, beispielsweise aus einem metallischen Werkstoff, oder auch als als Kunststofffaden ausgebildet sein. Das Führungselement 40b weist ein distales Ende 44b sowie ein proximales Ende 46b auf. An dem distalen Ende 44b ist eine Verdickung 48b als Begrenzung ausgebildet.

Weiterhin umfasst die Detektionsvorrichtung 1b ein Stabilisierungselement 60b, welches zur Stabilisierung und Fixierung des Führungselements 40b angeordnet ist. Das Stabilisierungselement kann eine Hülse 61b umfassen, durch die das Führungselement 40b hindurchgeführt ist. Die Hülse 61b kann ferner über ihr proximales Ende 64b mit einer Druck-Zug-Vorrichtung 68b verbunden sein. Dabei kann das Führungselement 40b über das proximale Ende 46b mit der Druck-Zug-Vorrichtung 68b verbunden sein, um so mittels der Druck-Zug-Vorrichtung 68b relativ zur Hülse 61b bewegt zu werden. Die Hülse 61b weist an ihrem distalen Ende 66b ferner ein Luer-Lock-Gewinde zur Verbindung mit Verweilkanülen auf.

Die Detektionsvorrichtung 1b umfasst ferner wenigstens zwei Funktionselemente 50b, die auf das Führungselement 40b aufgeschoben beziehungsweise aufgesteckt sind. Vorzugsweise sind mehr als zwei Funktionselemente 50b auf das Führungselement 40b aufgeschoben oder aufgesteckt. Bei den Funktionselementen kann es sich um Ringsegmente 50b handeln, welche beispielsweise kugel- oder knochenförmig ausgebildet sein können. In dem in Fig. 3 gezeigten Längsquerschnitt weisen die Funktionselemente eine Knochenform auf. In einem Zustand, in dem die Funktionselemente 50b auf das Führungselement 40b aufgeschoben oder aufgesteckt sind, erfährt dieses eine Stabilisierung, ohne dass die Flexibilität vollständig verloren geht.

Zum Aufschieben oder Aufstecken der Funktionselemente 50b auf das Führungselement 40b muss dieses von der Druck-Zug-Vorrichtung 68b getrennt und außerhalb der Hülse 61b befindlich sein. Die Funktionselemente 50b können dementsprechend vom proximalen Ende 46b des Führungselements 40b auf dieses aufgeschoben oder aufgesteckt werden. Das zuerst aufgeschobene Funktionselement 50b kommt an der Begrenzung 48b am distalen Ende 44b des Führungselementes 40b zur Anlage. Jedes weitere Funktionselement 50b, welches auf das Funktionselement 40b aufgeschoben wird, kann an dem jeweils zuvor aufgeschobenen Funktionselement 50b zur Anlage kommen. Nach Anordnung der jeweils benötigten Anzahl von Funktionselementen 50b kann das Führungselement 40b über sein proximales Ende 46b in die Hülse 61b hineingeschoben werden. Das proximale Ende 46b des Führungselements 40b kann durch das proximale Ende 64b der Hülse 61b wieder aus dieser herausgeführt werden, um mit der Druck-Zug-Vorrichtung 68b verbunden zu werden. Diese kann im Anschluss mit dem proximalen Ende 64b der Hülse 61b verbunden werden. Die Funktionselemente 50b können innerhalb der Hülse 61b aufbewahrt werden.

Für die in vivo und/oder in vitro Anreicherung von Probenmaterial mittels der Detektionsvorrichtung 1b wird beispielsweise eine an das distale Ende 66b der Hülse 61b angeschlossene Verweilkanüle in einen menschlichen Körper eingeführt. Mittels der Druck-Zug-Vorrichtung 68b kann nun das Führungselement 40b samt Funktionselementen 50b aus der Hülse 61b in die jeweilige Körperöffnung appliziert und anschließend wieder in die Hülse 61b hineinbewegt werden.

Um nach der in vivo und/oder in vitro Anreicherung von Probenmaterial mittels der Detektionsvorrichtung 1b die einzelnen Funktionselemente 50b für nachgeordnete Diagnoseverfahren zur Verfügung zu stellen, wird zunächst die Druck-Zug-Vorrichtung 68b von der Hülse 61b gelöst, dann das Führungselement von der Druck-Zug-Vorrichtung 68b entfernt und aus der Hülse 61b herausgezogen. Die Funktionselemente 50b können anschließend einzeln vom Führungselement 40b abgenommen werden.

Jedes der Funktionselemente 50b weist eine mit Detektionsrezeptoren 12 bestückte Funktionsfläche 10 auf, wobei auf die vorangehende Beschreibung des ersten Ausführungsbeispiels verwiesen wird.

### Drittes Ausführungsbeispiel

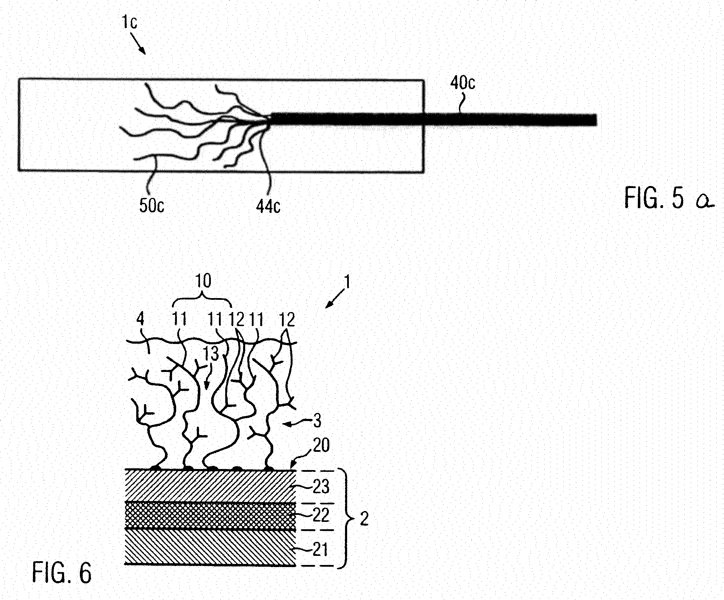

Figur 5a ist eine schematische Darstellung eines Detektionskatheters 1c gemäß einem dritten Ausführungsbeispiel. Auch bei der der erfindungsgemäßen Detektionsvorrichtung 1c nach dem zweiten Ausführungsbeispiel handelt es sich um einen biofunktionalisierten, medizinischen Detektionskatheter für die invasive (in vivo) Anreicherung von seltenen Zellen, Biomolekülen oder Arzneimitteln. Wie voranstehend bereits erläutert, wird ein derartiger Detektionskatheter beziehungsweise Detektor auch als medizinscher Nano-Katheter (MN-K) bzw. medical nano-catheter (MN-C) bezeichnet.

Die erfindungsgemäße Detektionsvorrichtung 1c nach dem dritten Ausführungsbeispiel umfasst einen flexiblen, medizinischen Führungsdraht 40c, mit einem distalen Ende 44c sowie einem proximalen Ende. Die Detektionsvorrichtung 1c nach dem dritten Ausführungsbeispiel kann ferner mit einem Stabilisierungselement in Form einer Hülse (hier nicht dargestellt) sowie einer Zug-/Druckvorrichtung (hier nicht dargestellt) ausgestattet sein. Insoweit wird auf das zweite Ausführungsbeispiel sowie die zugehörigen Figuren Bezug genommen.

Die Detektionsvorrichtung 1c umfasst ferner wenigstens zwei Funktionselemente 50c, die als Fäden ausgebildet sind. Die Fäden 50c sind gemäß dem dritten Ausführungsbeispiel am freien distalen Ende 44c des Führungsdrahtes 40c fest mit diesem verbunden. Vorzugsweise haben die Fäden 50c eine geringere Dicke als der Führungsdraht 40c. Die Dicke der Fäden 50c ist insbesondere so zu wählen, dass im Einsatz keine Thrombosen verursacht werden. Gleichzeitig ist die Dicke der Fäden so zu wählen, dass diese nicht verkleben, sondern sich im Blutstrom gleichmäßig verteilen.

Die Flexibilität der Fäden 50c kann im Einsatz einer Detektionsvorrichtung 1c gewährleisten, dass der gesamte Venenquerschnitt abgedeckt wird und darüber hinaus eine Bewegungsfreiheit in Längsrichtung sowie quer zur Längsrichtung der jeweiligen Vene gegeben ist. Die Fäden 50c bilden somit im Einsatz eine Struktur nach Art eines "Tentakels". Die hierdurch erreichte Abdeckung des gesamten Venenquerschnitts stellen eine gute Anreichung von Probenmaterial aus dem Blut sicher.

Um nach der in vivo und/oder in vitro Anreicherung von Probenmaterial mittels der Detektionsvorrichtung 1c die einzelnen als Fäden 50c ausgebildeten Funktionselemente für nachgeordnete Diagnoseverfahren zur Verfügung zu stellen, können die Fäden 50c einzeln von dem Führungsdraht 40c abgeschnitten oder abgerissen werden.

Jedes der Funktionselemente 50c weist eine mit Detektionsrezeptoren 12 bestückte Funktionsfläche 10 auf, wobei auf die vorangehende Beschreibung des ersten oder zweiten Ausführungsbeispiels verwiesen wird.

### Viertes Ausführungsbeispiel

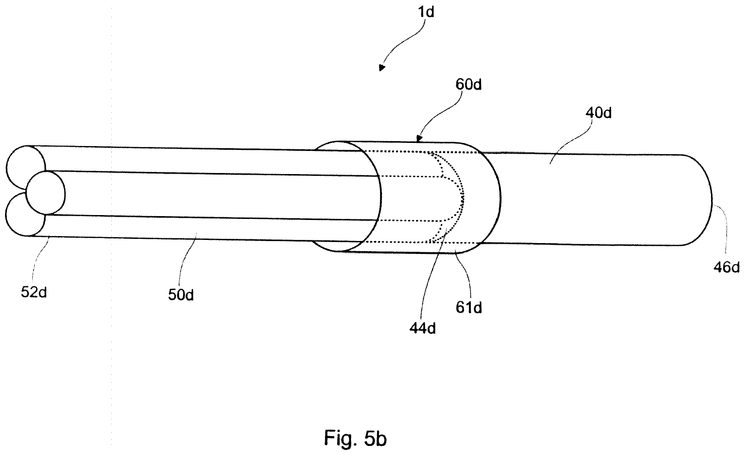

Figur 5b ist eine schematische Darstellung eines Detektionskatheters 1d gemäß einem vierten Ausführungsbeispiel. Auch bei der der erfindungsgemäßen Detektionsvorrichtung 1c nach dem zweiten Ausführungsbeispiel handelt es sich um einen biofunktionalisierten, medizinischen Detektionskatheter für die invasive (in vivo) Anreicherung von seltenen Zellen, Biomolekülen oder Arzneimitteln. Wie voranstehend bereits erläutert, wird ein derartiger Detektionskatheter beziehungsweise Detektor auch als medizinscher Nano-Katheter (MN-K) bzw. medical nano-catheter (MN-C) bezeichnet.

Die erfindungsgemäße Detektionsvorrichtung 1d nach dem vierten Ausführungsbeispiel umfasst einen flexiblen, medizinischen Führungsdraht 40d, mit einem distalen Ende 44d sowie einem proximalen Ende 46d. Die Detektionsvorrichtung 1d nach dem vierten Ausführungsbeispiel kann ferner mit einer Zug-/Druckvorrichtung (hier nicht dargestellt) ausgestattet sein. Insoweit wird auf das zweite Ausführungsbeispiel sowie die zugehörigen Figuren Bezug genommen.

Die Detektionsvorrichtung 1d umfasst ferner wenigstens zwei Funktionselemente 50d, vorzugsweise mehr als zwei Funktionselemente 50d, die als Drähte ausgebildet sind. Die Drähte 50d sind gemäß dem vierten Ausführungsbeispiel mit dem freien distalen Ende 44d des Führungsdrahtes 40d fest verbunden, insbesondere verschweißt. Die Detektionsvorrichtung 1d kann weiterhin mit einem Stabilisierungselement 60d in Form einer Hülse ausgestattet sein, die vorzugsweise die Verbindungsstelle zwischen den Drähten 50d sowie dem distalen Ende 44d des Führungsdrahtes 40d abdeckt, insbesondere umgibt. Weiterhin sind die Drähte 50d an ihren distalen Enden 52d miteinander verbunden, insbesondere miteinander verschweißt, sodass die Drähte 50d insgesamt ein Bündel von Funktionselementen 50d bilden.

Vorzugsweise haben die Drähte 50d jeweils eine geringere Dicke als der Führungsdraht 40d. Die Dicke der Drähte 50d ist insbesondere so zu wählen, dass im Einsatz keine Thrombosen verursacht werden. Gleichzeitig ist die Dicke der Drähte 50d so zu wählen, dass diese im Bündel gut durch den Führungsdraht 40d geführt werden können.

Um nach der in vivo und/oder in vitro Anreicherung von Probenmaterial mittels der Detektionsvorrichtung 1d die einzelnen als Drähte 50d ausgebildeten Funktionselemente für nachgeordnete Diagnoseverfahren zur Verfügung zu stellen, können die Drähte 50d einzeln von dem Führungsdraht 40d abgeschnitten oder abgerissen werden. Zudem besteht die Möglichkeit, die zwischen den Drähten 50d an ihren Enden 52d vorliegende Verbindung, bei der es sich beispielsweise um eine Schweißverbindung handeln kann, durch reißen oder schneiden zu lösen, um schließlich eine Vereinzelung der Drähte 50d zu erzielen.

Jedes der Funktionselemente 50d weist eine mit Detektionsrezeptoren 12 bestückte Funktionsfläche 10 auf, wobei auf die vorangehende Beschreibung des ersten oder zweiten Ausführungsbeispiels verwiesen wird.

### Funktionsfläche und Träger der Funktionsfläche

Erfindungsgemäß kann die Funktionsfläche 10 an einem Träger 2 ausgebildet sein. Dabei kann der Träger 2 Teil eines Funktionselements 50a, 50b oder 50c sein. Ein beispielhafter Schichtaufbau eines Trägers 2 ist in Figur 6 gezeigt.

Ein Substrat 21 aus medizinischem Edelstahldraht mit einem Durchmesser von ca. 0,5 mm verleiht dem Träger 2 seine sichtbare Struktur. Das Substrat 21 kann eine oder mehrere Beschichtungen 22, 23 aufweisen. Vorzugsweise ist das Substrat 21 mit einer Goldbeschichtung 22 mit eine Dicke von 0,5 bis 1,0 µm überzogen, die durch galvanische Verfahren, keramische Verfahren, Zementation oder durch Bedampfung aufgebracht wird. Das Substrat 21 kann ferner mit einem biokompatiblen Farbstoff beschichtet sein, um Eigenfluoreszenz des Grundmaterials während mikroskopischer Auswertung zu verringern.

Die chemische Aktivierung des Trägers 2 erfolgt über eine Affinitätsreaktion meist durch schwefel- oder stickstoffhaltige Verbindungen, an denen wiederum direkt oder über Polymerketten spezifische Detektionsrezeptoren 12 gebunden werden können.

Vorzugsweise wird auf dem Träger 2 über nasschemische oder physikalische Verfahren eine kovalente Sekundärschicht bestehend aus einem funktionalen biokompatiblen Polymer 3 aufgetragen. Die Schichtdicke kann 1 bis 2 µm betragen. Dadurch wird der Träger 2 chemisch aktiviert. Durch die Oberflächenveredelung und chemische Aktivierung lassen sich spezifische Antikörper, insbesondere monoklonale Antikörper murinen Ursprungs, chimäre Antikörper, humanisierte Antikörper, oder Fragmente dieser Antikörper oder Aminosäure-Strukturen oder Nukleinsäure-Strukturen oder synthetische Strukturen mit spezifischer Affinität zu Zelloberflächen oder Molekülen als Detektionsrezeptoren 12 kovalent anbinden.

Gerade bei einer komplexen Probenflüssigkeit wie Blut ist nicht nur eine dauerhafte Anbindung der Detektionsrezeptoren 12 unter Erhalt der biologischen Funktion, sondern auch eine effiziente Unterdrückung unspezifischer Adsorptionsprozesse für die selektive Anbindung der Liganden von entscheidender Bedeutung.

Eine Zwischenschicht 23 hat hierbei die Aufgabe, eine effektive Abschirmung der Oberfläche des Substrats 21 zu gewährleisten und gleichzeitig die funktionellen Gruppen für die Bindung der biokompatiblen Polymerschicht 3 in ausreichender Dichte zur Verfügung zu stellen. Das Zwischenschichtsystem bildet demnach einen Haftvermittler zwischen der Goldbeschichtung 22 des Substrats 21 und der biokompatiblen Polymerschicht 3.

Das biokompatible Polymer 3 ist vorzugsweise ein Hydrogel mit kohlenstoffhaltigen langen verzweigten Makromolekülen, welche über eine hohe Anzahl an funktionellen Gruppen, z.B. Carboxyl-Gruppen bzw. Polycarboxylaten, verfügen. Die Art der funktionellen Gruppen richtet sich nach den Moleküleigenschaften der spezifischen Detektionsrezeptoren 12. Das biokompatible Hydrogel gewährleistet dadurch die dauerhafte kovalente Bindung der Detektionsrezeptoren 12 unter Erhalt der biologischen Funktion und verhindert gleichzeitig, dass die Detektionsrezeptoren 12 durch unspezifische Adsorptionsphänomene in ihrer Erkennungsfunktion beeinträchtigt werden. Hydrogele sind dreidimensional vernetzte hydrophile Polymere, die Flüssigkeiten wie z.B. Wasser aufnehmen, selbst aber darin unlöslich sind. Hauptbestandteile des Hydrogels sind Polyacrylsäure (PAA) und Polyethylenglycol (PEG). Über eine geeignete Auswahl der Monomerbausteine, des Vernetzungsgrades und der Vernetzungsdichte lassen sich Eigenschaftsprofile je nach gewünschten Anforderungen oder Einsatzbereiche maßschneidern. Eine wesentliche Eigenschaft ist die Biokompatibilität, d. h. die Verträglichkeit des Hydrogels mit dem lebenden Gewebe. Durch die verzweigten Polymerketten des biokompatiblen Polymers 3 wird aber auch die thrombogene Wirkung während der invasiven Anwendung unterbunden. Durch chemische Aktivierung erhalten die funktionellen Gruppen eine unausgeglichene Molekülladung, die es ermöglicht, gelöste Detektionsrezeptoren 12 aus einer Lösung elektrostatisch anzuziehen und kovalent zu binden. Die dauerhaft an der Polymerschicht 3 immobilisierten Detektionsrezeptoren 12 dienen der spezifischen Bindung der Liganden bzw. Zielmoleküle und Zielzellen über ihre Oberflächenantigene und ermöglichen so die Funktion der Detektionsvorrichtung 1. Zusätzlich können in diesem biokompatiblen Polymer 3 chemisch oder enzymatisch spaltbare Gruppen enthalten sein, um die quantitative Gewinnung von gebundenen Zielmolekülen oder Zellen zu vereinfachen.

Die verzweigten Molekülstrukturen des biokompatiblen Polymers 3 bilden eine im mikroskopischen Bereich dreidimensional strukturierte Funktionsfläche 10 mit einander zugewandten Funktionsabschnitten 11 und von Probenflüssigkeit durchsetzbaren Zwischenräumen 13. Während die im makroskopisch bzw. sichtbaren Bereich dreidimensional strukturierte Oberfläche des Trägers 2 (Figur 6) die Probenflüssigkeit in vorteilhafter Weise über das jeweilige Funktionselement 50a, 50b, 50c leitet, verlangsamt die im mikroskopischen Bereich dreidimensional strukturierte Funktionsfläche 10 des biokompatiblen Polymers 3 (vgl. Figur 6) die Strömung der Probenflüssigkeit im Bereich der Grenzschicht und begünstigt die Anreicherung der Liganden an den Detektionsrezeptoren 12.

Zum Konservieren und zum Schutz vor den Bedingungen der Endsterilisation sowie zum Strahlenschutz und zur Haltbarkeit des Produkts wird über das biokompatible Polymer 3 eine biokompatible Schutzschicht (tertiäre Schicht bzw. Stabilisatorschicht) 4 aufgebracht. Diese Schutzschicht 4 trocknet über der Sekundärschicht ein und bildet ein dichtes Netz aus kristallinen Strukturen und stabilisiert und konserviert somit den funktionellen Teil 1a des Katheters 1. Die Schutzschicht 4 ist nicht kovalent gebunden. Im Blutstrom geht die Schutzschicht 4 in Lösung und gibt die Funktionsfläche 10 des Katheters frei. Alternativ kann die Schutzschicht 4 vor der Anwendung mit sterilem Wasser abgewaschen werden.

Die Schutzschicht 4 kann hochreine Alginate, Polyethylenglykole, zyklische und nicht zyklische Oligosaccharide und Polysaccharide, antioxidative Aminosäuren, Proteine und Vitamine umfassen. Die Schutzschicht 4 besteht vorzugsweise aus einem biokompatiblen hochviskosen Polysaccharid, welches als Medium für zugesetzte Aminosäuren, Proteinen, Vitaminen und stabilisierende Polysaccharide dient. Die hohe Viskosität erlaubt eine rasche Benetzbarkeit der Oberfläche. Die angelagerte Schutzschicht 4 klebt an der Sekundärbeschichtung und verhindert das Eindringen von Fremdsubstanzen während einer Lagerung. Die zugesetzten Aminosäuren, Proteine und Vitamine sind im Vergleich zu den spezifischen Liganden in höheren Konzentrationen vorhanden und dadurch in der Lage, die Wahrscheinlichkeit einer Schädigung der Zielmoleküle durch radikale Moleküle oder Ladungsträger auf sich zu lenken bzw. abzufangen und durch Rekombinationsprozesse zerstörte chemische Bindungen wiederherzustellen.

Die fertig gestellte Detektionsvorrichtung 1a, 1b, 1c wird in keimarmer Umgebung verpackt. Die Endsterilisation erfolgt mittels Gamma-Bestrahlung bei einer Strahlungsdosis von 25 kGy. Die Detektionsvorrichtung 1a, 1b, 1c ist für die einmalige Anwendung vorgesehen.

### Anwendung einer erfindungsgemäßen Detektionsvorrichtung

Die erfindungsgemäß hergestellte Detektionsvorrichtung 1a, 1b, 1c mit veredelter Funktionsfläche 10 und mit gekoppelten Detektionsrezeptoren 12 eignet sich für die Gewinnung seltener Zellen aus dem Blutkreislauf. Hierzu zählen beispielsweise folgende Anwendungen:
- Gewinnung von embryonalen Trophoblasten aus dem mütterlichen Blutkreislauf mit z.B. spezifischen Antikörperfragmenten (F(ab)-Fragmenten) und murinen monoklonalen Antikörpern (IgG), welche das für Trophoblasten typische Zelloberflächenprotein HLA-G erkennen können.
- Gewinnung von disseminierten Tumorzellen, insbesondere von hämatogen metastasierenden Tumoren z.B. mit dem humanisierten Antikörper Anti-EpCAM, welcher das für viele Krebszellen typische Zelloberflächenprotein EpCAM erkennt.

Eine bevorzugte Anwendung der Detektionsvorrichtung 1a, 1b, 1c liegt in der pränatalen und Krebs-Diagnostik. Die Detektionsvorrichtung 1a, 1b, 1c kann beispielsweise dafür eingesetzt werden, im Blutkreislauf von Schwangeren oder Krebspatienten zirkulierende fetale Zellen oder Tumorzellen zu isolieren. Zur Anwendung wird die Detektionsvorrichtung 1a, 1b, 1c über ein geeignetes kommerziell erhältliches Braunülensystem in die Vene eingeführt und in den venösen Blutkreislauf appliziert. Die Verweildauer in der Vene kann ca. 30 min betragen. Nach der Entnahme der Detektionsvorrichtung 1a, 1b, 1c aus der Blutbahn werden die auf der Detektionsvorrichtung 1a, 1b, 1c gebundenen Zellen mittels gezielter Labordiagnostik weiter angereichert und molekularbiologisch sowie zellbiologisch charakterisiert.

Ziel des durchzuführenden minimal invasiven Verfahrens ist die Selektion von fetalen oder Tumor-Zellen aus dem Blut. Auf Grund der niedrigen Zellkonzentration der Zellen im Blut wäre eine Blutentnahme von ca. 0,5 I notwendig, um die gewünschte Zielzellzahl zu erhalten. Dies ist aus medizinischer Sicht jedoch ausgeschlossen.

Bei der pränatalen Diagnostik soll eine eventuelle Chromosomenaberration (z. B. Trisomie 21 (Down-Syndrom)) mit Hilfe der im Blut der Mutter enthaltenen fetalen Zellen nachgewiesen werden. Das Down-Syndrom wird pränatal bislang sicher nur durch invasive Verfahren, die jeweils ein Abortrisiko von 1 % aufweisen, diagnostiziert: Chorionzottenbiopsie zwischen der 11. und 14. Schwangerschaftswoche und Amniozentese (Fruchtwasseruntersuchung) ab der 15. Schwangerschaftswoche. Das erfindungsgemäße Verfahren hingegen, das ab der 9. Schwangerschaftswoche einsetzbar sein wird, weist kein Risiko für den Föten auf und kann im Erst-Trimester-Screening eingesetzt werden. Somit kann auf die Amniozentesen verzichten werden.

Fetale Trophoblastenzellen aus der Plazenta können ab der 6. Schwangerschaftswoche im Blutkreislauf der Mutter nachgewiesen werden. Es kommen nur ca. 2 - 5 dieser Zellen pro ml Mutterblut vor. Diese Trophoblastenzellen besitzen einen membrangebundenen HLA-G Komplex (Antigen), der an bestimmte Antikörper bindet. Vorzugsweise wird ein spezifischer HLA-G Antikörper als Detektionsrezeptor 12 eingesetzt, der nur mit membrangebundenem HLA-G (Antigen) reagiert und somit nur die gewünschten fetalen Zellen aus dem Mutterblut fangen soll.

Krebstumorzellen können mit dem EpCAM Antikörper (gegen das EpCAM Antigen) angereichert werden, der in seinen konstanten Domänen humanisiert ist und kovalent and das Hydrogel gebunden wird.

## Patentansprüche

1. Detektionsvorrichtung (1a, 1b, 1c, 1d) zur in vivo und/oder in vitro Anreicherung von Probenmaterial, mit wenigstens einem Führungselement (40a, 40b, 40c, 40d) zur Führung zumindest eines Funktionselements (40a, 40b, 40c, 40d) und mit zumindest zwei an dem Führungselement (40a, 40b, 40c, 40d) angeordneten Funktionselementen (50a, 50b, 50c, 50d), an denen jeweils eine mit Detektionsrezeptoren (12) bestückte Funktionsfläche (10) ausgebildet ist, wobei die Funktionselemente (50a, 50b, 50c, 50d) voneinander lösbar ausgebildet und/oder einzeln von dem Führungselement (40a, 40b, 40c, 40d) lösbar sind, **dadurch gekennzeichnet, dass** zumindest ein als ringförmiges Segment ausgebildetes Funktionselement (50a, 50b) auf das Führungselement (40a, 40b) aufgeschraubt ist und/oder dass zumindest ein als flexibler Draht oder Faden ausgebildetes Funktionselement (50c, 50d) an dem distalen Ende (44c, 44d) des Führungselements (40c, 40d) stoffschlüssig befestigt ist.

2. Detektionsvorrichtung (1a, 1b, 1c, 1d) nach wenigstens einem der vorangegangenen Ansprüche, **dadurch gekennzeichnet, dass** das Führungselement (40a, 40b, 40c, 40d) wenigstens eine der folgenden Anforderungen erfüllt:
a. Das Führungselement (40a, 40b, 40c, 40d) ist zumindest abschnittsweise als Draht ausgebildet.
b. Das Führungselement (40a, 40b, 40c, 40d) ist zumindest abschnittsweise federelastisch ausgebildet.
c. Das Führungselement (40a, 40b, 40c, 40d) ist zumindest abschnittsweise als flexibler medizinischer Führungsdraht ausgebildet.
d. Das Führungselement (40a, 40b, 40c) ist zumindest abschnittsweise als Faden ausgebildet.
e. Das Führungselement (40a, 40b, 40c) ist zumindest abschnittsweise als flexibler Kunststofffaden ausgebildet.
f. Das Führungselement (40a, 40b, 40c) ist zumindest abschnittsweise als Katheter ausgebildet.
g. Das Führungselement (40a, 40b) weist einen Aufnahmeabschnitt zur Aufnahme zumindest eines Funktionselements (50a, 50b) auf.
h. Das Führungselement (40a, 40b) weist ein distales Ende (44a, 44b) sowie ein proximales Ende (46a, 46b) auf, wobei zwischen dem distalen und dem proximalen Ende ein Aufnahmeabschnitt zur Aufnahme zumindest eines Funktionselements (50a, 50b) ausgebildet ist.
i. Das Führungselement (40a, 40b, 40c) ist zumindest abschnittsweise schraubenförmig ausgebildet.
j. Das Führungselement (40a) weist an seinem Außenumfang (42a) zumindest abschnittsweise ein Außengewinde auf.
k. Das Führungselement (40a, 40b, 40c, 40d) weist ein distales Ende (44a, 44b, 44c, 44d) sowie ein proximales Ende (46a, 46b, 46c, 46d) auf, wobei das distale Ende (44a, 44b, 44c, 44d) in ein Blutgefäß einführbar ist.
l. Das Führungselement (40a, 40b) weist ein distales Ende (44a, 44b) sowie ein proximales Ende (46a, 46b) auf, wobei das distale Ende (44a, 44b) verdickt ausgebildet ist.
m. Das Führungselement (40a) weist ein distales Ende (44a) sowie ein proximales Ende (46a) auf, wobei das proximale Ende (46a) mit einem Stabilisierungselement (60a) verbunden ist.
n. Das Führungselement (40a) ist in ein Innengewinde des Stabilisierungselements (60a) eingeschraubt.
o. Das Führungselement (40a, 40b, 40c, 40d) ist aus einem metallischen und/oder nicht-metallischen Material gefertigt.
p. Das Führungselement (40a, 40b, 40c, 40d) weist an seinem distalen Abschnitt eine mit Detektionsrezeptoren (12) bestückte Funktionsfläche (10) auf.

3. Detektionsvorrichtung (1a, 1b, 1c, 1d) nach wenigstens einem der vorangegangenen Ansprüche, **dadurch gekennzeichnet, dass** das Führungselement (40a, 40b, 40c, 40d) mit einem Stabilisierungselement (60a, 60b, 60d) zur Stabilisierung des wenigstens einen Führungselements (40a, 40b, 40c, 40d) verbunden ist, welches vorzugsweise wenigstens eine der folgenden Anforderungen erfüllt:
a. Das Stabilisierungselement (60a, 60b, 60d) ist dazu ausgebildet, das Führungselement (40a, 40b, 40d) zumindest abschnittsweise zu stabilisieren.
b. Das Stabilisierungselement (60a, 60b, 60d) ist aus Kunststoff oder Metall gefertigt.
c. Das Stabilisierungselement (60a, 60b, 60d) ist mit einem proximalen Ende (46a, 46b, 46d) oder einem proximalen Endabschnitt (46a, 46b, 46d) des Führungselements (40a, 40b, 40d) verbunden.
d. Das Stabilisierungselement (60a, 60b, 60d) ist lösbar mit dem Führungselement (40a, 40b, 40d) verbunden.
e. Das Stabilisierungselement (60a, 60b, 60d) ist form-, kraft- und/oder stoffschlüssig mit dem Führungselement (40a, 40b, 40d) verbunden.
f. Das Stabilisierungselement (60a, 60b, 60d) ist mit dem Führungselement (40a, 40b, 40d) verklebt oder verschweißt.
g. Das Stabilisierungselement (60a, 60b, 60d) ist zumindest abschnittsweise zylindrisch ausgebildet.
h. Das Stabilisierungselement (60a, 60b, 60d) ist zumindest abschnittsweise als Hülse (60a, 61b, 61d) ausgebildet.
i. Das Stabilisierungselement (60a, 60b, 60d) weist zumindest abschnittsweise ein Innengewinde auf.
j. Das Stabilisierungselement (60a, 60b) ist zumindest abschnittsweise auf einen proximalen Abschnitt des Führungselementes (40a, 40b) aufgeschoben, aufgesteckt oder aufgeschraubt.
k. Das Stabilisierungselement (60d) ist zumindest abschnittsweise auf einen distalen Abschnitt (44d) des Führungselementes (40d) aufgeschoben, aufgesteckt oder aufgeschraubt.
l. Das Stabilisierungselement (60a, 60b, 60d) führt das Führungselement (40a, 40b, 40d) zumindest abschnittsweise.
m. Das Stabilisierungselement (60a, 60b, 60d) führt das Führungselement (40a, 40b, 40d) beweglich.
n. Das Stabilisierungselement (60b, 60d) ist als Abdeckvorrichtung für zumindest ein Funktionselement (50b, 50d) ausgebildet.
o. Das Stabilisierungselement (60b, 60d) weist eine Aufnahme auf, in der die zumindest zwei Funktionselemente (50b, 50d) angeordnet sind.
p. Das Stabilisierungselement (60b, 60d) ist in ein Blutgefäß einführbar.
q. Das Stabilisierungselement (60b, 60d) weist eine Aufnahme auf, in der die zumindest zwei Funktionselemente (50b, 50d) herausführbar angeordnet sind.
r. Das Stabilisierungselement (60b) weist ein distales Ende (66b) und ein proximales Ende (64b) auf, wobei an dem distalen Ende (66b) ein Gewinde, insbesondere ein Luer-Lock-Gewinde zum Anschließen von Verweilkanülen, ausgebildet ist.
s. Das Stabilisierungselement (60a, 60b) weist eine Druck-Zug-Vorrichtung (68b) auf, mittels der die zumindest zwei Funktionselemente (50b) aus der Hülse (61b) heraus- und wieder in diese hineinführbar sind.
t. Das Stabilisierungselement (60a, 60b) weist zumindest einen Abschnitt auf, dessen Aussendurchmesser dem Aussendurchmesser des distalen Abschnitts (44a, 44b) des Führungselements (40a, 40b) entspricht oder im Wesentlichen entspricht.
u. Das Stabilisierungselement (60a, 60b) ist mit dem Führungselement (40a, 40b) in einem Herstellverfahren gefertigt.
v. Das Stabilisierungselement (60a, 60b, 60d) weist ein abgerundetes Ende als Verletzungsschutz auf.
w. Das Stabilisierungselement (60d) ist zur Stabilisierung der Verbindungsstelle zwischen zumindest einem der Funktionselemente (50d) sowie dem Führungselement (40d) ausgebildet.

4. Detektionsvorrichtung (1a, 1b, 1c, 1d) nach wenigstens einem der vorangegangenen Ansprüche, **dadurch gekennzeichnet, dass** eine Abdeckvorrichtung (61b, 61d) vorgesehen ist, die wenigstens eine der folgenden Anforderungen erfüllt:
a. Die Abdeckvorrichtung (61b, 61d) ist zur Abdeckung zumindest eines der Funktionselemente (50a, 50b, 50c, 50d) ausgebildet.
b. Die Abdeckvorrichtung (61b, 61d) ist Bestandteil des Stabilisierungselements (60a, 60b, 60d).
c. Die Abdeckvorrichtung (61b, 61d) ist als von dem Stabilisierungselement (60a, 60b, 60d) diskrete Komponente ausgebildet.
d. Die Abdeckvorrichtung (61b, 61d) ist dazu ausgebildet, das Führungselement (40a, 40b, 40c, 40d) zumindest abschnittsweise zu stabilisieren.
e. Die Abdeckvorrichtung (61b, 61d) ist aus Kunststoff oder Metall gefertigt.
f. Die Abdeckvorrichtung (61b, 61d) ist zumindest abschnittsweise zylindrisch ausgebildet.
g. Die Abdeckvorrichtung (61b, 61d) weist eine longitudinale Bohrung auf, zur Aufnahme zumindest eines Funktionselements (50a, 50b, 50c, 50d).
h. Die Abdeckvorrichtung (61b, 61d) ist zumindest abschnittsweise als Hülse ausgebildet.
i. Die Abdeckvorrichtung (61b, 61d) ist zumindest abschnittsweise auf einen proximalen Abschnitt des Führungselementes (40a, 40b, 40c, 40d) aufgeschoben, aufgesteckt oder aufgeschraubt.
j. Die Abdeckvorrichtung (61b, 61d) ist in ein Blutgefäß einführbar.
k. Die Abdeckvorrichtung (61b, 61d) weist eine Aufnahme auf, in der die zumindest zwei Funktionselemente (50a, 50b, 50d) herausführbar angeordnet sind.
l. Die Abdeckvorrichtung (61b, 61d) ist entlang einer Längsorientierung relativ zu den Funktionselementen (50a, 50b, 50c, 50d) und/oder relativ zum Stabilisierungselement (60a, 60b, 60d) beweglich angeordnet.
m. Die Abdeckvorrichtung (61b) weist ein distales Ende (66b) und ein proximales Ende (64b) auf, wobei an dem distalen Ende (66b) ein Gewinde, insbesondere ein Luer-Lock-Gewinde zum Anschließen von Verweilkanülen, ausgebildet ist.
n. Die Abdeckvorrichtung (61b, 61d) weist Homopolymere, Copolymere, Biopolymere, chemisch modifizierte Polymere und/oder synthetische Polymere auf.
o. Die Abdeckvorrichtung (61b, 61d) weist ein abgerundetes Ende als Verletzungsschutz auf.
p. Die Abdeckvorrichtung (61d) ist zur Abdeckung der Verbindungsstelle zwischen zumindest einem der Funktionselemente (50d) sowie dem Führungselement (40d) ausgebildet.

5. Detektionsvorrichtung (1a, 1b, 1c, 1d) nach Anspruch 1, **dadurch gekennzeichnet, dass** zumindest eines der Funktionselemente (50a, 50b, 50c, 50d) wenigstens eine der folgenden Anforderungen erfüllt:
a. Das Funktionselement (50a, 50b, 50c, 50d) ist zumindest teilweise aus Metall, vorzugsweise Edelstahl, medizinischem Edelstahl oder Titan; aus Glas, vorzugsweise Glasfaser; aus Kunststoff, vorzugsweise einem geschäumten Kunststoff, einem Polymer, bevorzugt Polyethylen, Polypropylen, Polyurethan, Polytetrafluorethylen, einem auf organischen Polymeren basierenden Kunststoff oder einer Kombination dieser Materialien gefertigt.
b. Das Funktionselement (50c) ist an dem distalen Ende (44c) des Führungselements (40c), insbesondere an einer Querschnittsfläche des distalen Endes (44c), befestigt.
c. Das Funktionselement (50c) ist an dem Außenumfang des Führungselements (40c) befestigt, wobei die Länge des Funktionselements (50c) bevorzugt größer ist als der Abstand der Verbindungsstelle am Außenumfang des Führungselements (40c) vom distalen Ende (44c) des Führungselements (40c).
d. Das Funktionselement (50c) ist an seinem distalen Ende mit wenigstens einem weiteren Funktionselement (50c), vorzugsweise mit wenigstens zwei weiteren Funktionselementen (50c) verbunden, vorzugsweise stoffschlüssig verbunden.
e. Das Funktionselement (50d) ist an einer Querschnittsfläche des distalen Endes (44d), befestigt, insbesondere an der Querschnittsfläche des distalen Endes (44d) angeschweißt.
f. Das Funktionselement (50d) ist an seinem distalen Ende mit wenigstens einem weiteren Funktionselement (50d), vorzugsweise mit wenigstens zwei weiteren Funktionselementen (50d) verbunden, insbesondere stoffschlüssig verbunden, insbesondere verschweißt.
g. Das Funktionselement (50a, 50b) ist zerstörungsfrei von dem zumindest einen anderen Funktionselement (50a, 50b) lösbar und/oder zerstörungsfrei von dem Führungselement (40a, 40b) abnehmbar.
h. Das Funktionselement (50c, 50d) ist vom Führungselement (40c, 40d) abschneidbar oder abreißbar.
i. Das Funktionselement (50a) umfasst an dessen Innenumfangsfläche (52a) ein Innengewinde.
j. Das Funktionselement (50a, 50b) umschließt zumindest einen Abschnitt des Führungselements (40a, 40b).
k. Das Funktionselement (50a, 50b) ist zwischen einem distalen Ende (44a, 44b) sowie einem proximalen Ende (46a, 46b) des Führungselements (40a, 40b) an diesem angeordnet.
l. Das Funktionselement (50a, 50b, 50c) weist Detektionsrezeptoren (12) auf, die sich von den Detektionsrezeptoren (12) des jeweils anderen Funktionselements (50a, 50b, 50c) unterscheiden.
m. Das Funktionselement (50a, 50b, 50c) weist eine Kombination von Detektionsrezeptoren (12) auf, die sich von der Kombination von Detektionsrezeptoren (12) des jeweils anderen Funktionselements (50a, 50b, 50c) unterscheidet.
n. Das Funktionselement (50a, 50b) weist zumindest einen Abschnitt mit einem Aussendurchmesser auf, der dem Aussendurchmesser des distalen Abschnitts (44a, 44b) des Führungselements (40a, 40b) entspricht oder im Wesentlichen entspricht.
o. Das Funktionselement (50a, 50b, 50c) weist zumindest einen Abschnitt mit einem Aussendurchmesser auf, der größer oder kleiner ist als der distale Abschnitt (44a, 44b) des Führungselements (40a, 40b), insbesondere 0,01 mm bis 0,1 mm kleiner ist als der distale Abschnitt (44a, 44b) des Führungselements (40a, 40b).
p. Das Funktionselement (50a, 50b) ist mit Erhöhungen, Vertiefungen und/oder Verzweigungen ausgebildet.
q. Das Funktionselement (50a, 50b) umfasst eine Beschichtung (22) aus Metall, vorzugsweise aus einem Metall aus der 10. oder 11. Gruppe des Periodensystems der Elemente, bevorzugt aus Nickel, Kupfer, Palladium, Silber, Platin und/oder Gold.

6. Detektionsvorrichtung (1a, 1b, 1c, 1d) nach wenigstens einem der vorangegangenen Ansprüche, **dadurch gekennzeichnet, dass** die Funktionsfläche (10) zumindest eines Funktionselements (50a, 50b, 50c, 50d) wenigstens eine der folgenden Anforderungen erfüllt:
a. Die Funktionsfläche (10) weist eine dreidimensionale Struktur mit einander zugewandten Funktionsabschnitten (11) auf, die wenigstens einen von einer Probenflüssigkeit durchsetzbaren Zwischenraum (13) bilden, wobei der Zwischenraum (13) vorzugsweise wenigstens abschnittsweise kanalförmig ausgebildet ist, wobei bevorzugt mehrere Zwischenräume vorzugsweise ein verzweigtes Netz von Kanälen bilden.
b. Die Funktionsfläche (10) ist im makroskopischen und/oder im mikroskopischen Bereich dreidimensional strukturiert.
c. Die Funktionsfläche (10) ist mit Erhöhungen, Vertiefungen und/oder Verzweigungen ausgebildet.
d. Die Funktionsfläche (10) umfasst zumindest teilweise eine spiralförmige, schraubenförmige, schneckenförmige, wellenförmige, helikale, filamentöse, bürstenförmige, kammförmige, netzförmige, poröse, schwammförmige oder dergleichen Struktur.

7. Detektionsvorrichtung (1a, 1b, 1c, 1d) nach wenigstens einem der vorangegangenen Ansprüche, **dadurch gekennzeichnet, dass** die Detektionsrezeptoren (12) Antikörper, Antikörperfragmente, Aminosäuren-Strukturen, Nukleinsäure-Strukturen, anorganischen Materialen und/oder synthetische Strukturen mit spezifischer Affinität zu Zelloberflächen umfassen, vorzugsweise monoklonale Antikörper murinen Ursprungs, chimäre Antikörper oder humanisierte Antikörper, bevorzugt HLA-G und/oder EpCAM Antikörper.

8. Detektionsvorrichtung (1a, 1b, 1c, 1d) nach wenigstens einem der vorangegangenen Ansprüche, **dadurch gekennzeichnet, dass** zumindest eines der Funktionselemente (50a, 50b) ein biokompatibles Polymer (3) aufweist, das vorzugsweise wenigstens eine der folgenden Anforderungen erfüllt:
a. Das biokompatible Polymer (3) ist als zusammenhängende Polymerschicht ausgebildet.
b. Das biokompatible Polymer (3) weist eine dreidimensionale, vorzugsweise filamentöse und/oder poröse Struktur auf.
c. Das biokompatible Polymer (3) weist eine dreidimensionale, vorzugsweise filamentöse, und/oder poröse Oberfläche auf.
d. Das biokompatible Polymer (3) weist eine kohlenstoffhaltige, verzweigte Molekülstruktur auf.
e. Das biokompatible Polymer (3) ist vorzugsweise über funktionelle Gruppen mit dem Funktionselement (50a, 50b, 50c, 50d) wirkverbunden, bevorzugt durch chemische Bindung, besonders bevorzugt durch kovalente Bindung.
f. Das biokompatible Polymer (3) umfasst funktionelle Gruppen, vorzugsweise Carboxyl-Gruppen, wobei die funktionellen Gruppen vorzugsweise durch chemische Aktivierung eine unausgeglichene Molekülladung aufweisen, wobei die funktionellen Gruppen bevorzugt auf die Detektionsrezeptoren (12) abgestimmt sind.
g. Das biokompatible Polymer (3) weist hydrophile Eigenschaften auf und ist vorzugsweise ein Hydrogel.
h. Das biokompatible Polymer (3) umfasst chemisch und/oder enzymatisch spaltbare Gruppen.
i. Das biokompatible Polymer (3) umfasst gesättigte Atomgruppen und kovalent gebundene Liganden und Rezeptoren.
j. Das biokompatible Polymer (3) ist in einem Hohlraum des Funktionselement (50a, 50b) angeordnet.
k. Das biokompatible Polymer (3) ist vernetzt.
l. Das biokompatible Polymer (3) umfasst und/oder bildet die Funktionsfläche (10).

9. Detektionsvorrichtung (1a, 1b, 1c, 1d) nach wenigstens einem der vorangegangenen Ansprüche, **dadurch gekennzeichnet, dass** die zumindest eine Funktionsfläche (10) mit einer Schutzschicht (4) überzogen ist, wobei die Schutzschicht (4) vorzugsweise wenigstens eine der folgenden Anforderungen erfüllt:
a. Die Schutzschicht (4) ist in Flüssigkeiten, insbesondere in Körperflüssigkeiten, vorzugsweise in Blut, löslich.
b. Die Schutzschicht (4) ist biokompatibel.
c. Die Schutzschicht (4) ist organisch kristallin und umfasst wenigstens einen der folgenden Bestandteile: Alginate, vorzugsweise hochreine Alginate, Polyethylenglykole, zyklische und nicht zyklische Oligosaccharide, Polysaccharide, antioxidative Aminosäuren, Proteine oder Vitamine.

10. Verwendung einer Detektionsvorrichtung (1a, 1b, 1c, 1d) nach wenigstens einem der vorangegangenen Ansprüche zur in vitro Anreicherung von Proben.

## Claims

1. A detection device (1a, 1b, 1c, 1d) for in vivo and/or in vitro enrichment of sample material, comprising at least one guide element (40a, 40b, 40c, 40d) for guiding at least one functional element (40a, 40b, 40c, 40d) and at least two functional elements (50a, 50b, 50c, 50d) disposed on the guide element (40a, 40b, 40c, 40d), each functional element having formed thereon a functional surface (10) equipped with detection receptors (12), wherein the functional elements (50a, 50b, 50c, 50d) are configured such that they are detachable from one another and/or are individually detachable from the guide element (40a, 40b, 40c, 40d), **characterized in that** at least one functional element (50a, 50b), which is configured as an annular segment, is screwed onto the guide element (40a, 40b) and/or that at least one functional element (50c, 50d) configured as a flexible wire or thread is fixed to the distal end (44c, 44d) of the guide element (40c, 40d) by a material bond.

2. The detection device (1a, 1b, 1c, 1d) according to at least one of the preceding claims, **characterized in that** the guide element (40a, 40b, 40c, 40d) satisfies at least one of the following requirements:
a. The guide element (40a, 40b, 40c, 40d) is configured as a wire, at least in sections.
b. The guide element (40a, 40b, 40c, 40d) is configured to be resilient, at least in sections.
c. The guide element (40a, 40b, 40c, 40d) is configured as a flexible medical guide wire, at least in sections.
d. The guide element (40a, 40b, 40c) is configured as a thread, at least in sections.
e. The guide element (40a, 40b, 40c) is configured as a flexible plastic thread, at least in sections.
f. The guide element (40a, 40b, 40c) is configured as a catheter, at least in sections.
g. The guide element (40a, 40b) comprises an accommodating section for accommodating at least one functional element (50a, 50b).
h. The guide element (40a, 40b) comprises a distal end (44a, 44b) and a proximal end (46a, 46b), an accommodating section for accommodating at least one functional element (50a, 50b) being formed between said distal end and said proximal end.
i. The guide element (40a, 40b, 40c) is screw-shaped, at least in sections.
j. The guide element (40a) comprises an external thread on its outer periphery (42a), at least in sections.
k. The guide element (40a, 40b, 40c, 40d) comprises a distal end (44a, 44b, 44c, 44d) and a proximal end (46a, 46b, 46c, 46d), wherein the distal end (44a, 44b, 44c, 44d) is adapted to be inserted into a blood vessel.
l. The guide element (40a, 40b) comprises a distal end (44a, 44b) and a proximal end (46a, 46b), the distal end (44a, 44b) being increased in thickness.
m. The guide element (40a) comprises a distal end (44a) and a proximal end (46a), the proximal end (46a) being connected to a stabilization element (60a).
n. The guide element (40a) is screwed into an internal thread of the stabilization element (60a).
o. The guide element (40a, 40b, 40c, 40d) is made of a metallic and/or a non-metallic material.
p. The guide element (40a, 40b, 40c, 40d) comprises a functional surface (10) equipped with detection receptors (12) on its distal section.

3. The detection device (1a, 1b, 1c, 1d) according to at least one of the preceding claims, **characterized in that** the guide element (40a, 40b, 40c, 40d) is connected to a stabilization element (60a, 60b, 60d) for stabilizing the at least one guide element (40a, 40b, 40c, 40d), which preferably satisfies at least one of the following requirements:
a. The stabilization element (60a, 60b, 60d) is configured for stabilizing the guide element (40a, 40b, 40d), at least in sections.
b. The stabilization element (60a, 60b, 60d) is made of plastics or metal.
c. The stabilization element (60a, 60b, 60d) is connected to a proximal end (46a, 46b, 46d) or a proximal end section (46a, 46b, 46d) of the guide element (40a, 40b, 40d).
d. The stabilization element (60a, 60b, 60d) is releasably connected to the guide element (40a, 40b, 40d).
e. The stabilization element (60a, 60b, 60d) is connected to the guide element (40a, 40b, 40d) by a form-fit connection, a force-fit connection and/or by a material bond.
f. The stabilization element (60a, 60b, 60d) is glued or welded to the guide element (40a, 40b, 40d).
g. The stabilization element (60a, 60b, 60d) is cylindrical in shape, at least in sections.
h. The stabilization element (60a, 60b, 60d) is configured as a sleeve (60a, 61b, 61d), at least in sections.
i. The stabilization element (60a, 60b, 60d) has an internal thread, at least in sections.
j. The stabilization element (60a, 60b) is, at least in sections, pushed-onto, put-onto or screwed onto a proximal section of the guide element (40a, 40b).
k. The stabilization element (60d) is, at least in sections, pushed-onto, put-onto or screwed onto a distal section (44d) of the guide element (40d).
l. The stabilization element (60a, 60b, 60d) guides the guide element (40a, 40b, 40d), at least in sections.
m. The stabilization element (60a, 60b, 60d) guides the guide element (40a, 40b, 40d) in a movable manner.
n. The stabilization element (60b, 60d) is configured as covering device for at least one functional element (50b, 50d).
o. The stabilization element (60b, 60d) comprises an accommodation unit having arranged therein the at least two functional elements (50b, 50d).
p. The stabilization element (60b, 60d) is adapted to be inserted into a blood vessel.
q. The stabilization element (60b, 60d) comprises an accommodation unit having arranged therein the at least two functional elements (50b, 50d) such that they can be guided out.
r. The stabilization element (60b) comprises a distal end (66b) and a proximal end (64b), wherein a thread, in particular a Luer-Lock thread, is formed on the distal end (66b) for connecting indwelling cannulas.
s. The stabilization element (60a, 60b) comprises a push-pull device (68b) by means of which the at least two functional elements (50b) can be guided out of and back into the sleeve (61b).
t. The stabilization element (60a, 60b) comprises at least one section whose outer diameter corresponds or substantially corresponds to the outer diameter of the distal section (44a, 44b) of the guide element (40a, 40b).
u. The stabilization element (60a, 60b) and the guide element (40a, 40b) are fabricated in one manufacturing process.
v. The stabilization element (60a, 60b, 60d) has a rounded end to protect from injuries.
w. The stabilization element (60d) is configured for stabilizing the joint between at least one of the functional elements (50d) and the guide element (40d).

4. The detection device (1a, 1b, 1c, 1d) according to at least one of the preceding claims, **characterized in that** a covering device (61 b, 61d) is provided, which satisfies at least one of the following requirements:
a. The covering device (61b, 61d) is configured for covering at least one of the functional elements (50a, 50b, 50c, 50d).
b. The covering device (61b, 61d) is part of the stabilization element (60a, 60b, 60d).
c. The covering device (61b, 61d) is configured as a component discrete from the stabilization element (60a, 60b, 60d).
d. The covering device (61b, 61d) is configured for stabilizing the guide element (40a, 40b, 40c, 40d), at least in sections.
e. The covering device (61b, 61d) is made of plastics or metal.
f. The covering device (61b, 61d) is configured cylindrically, at least in sections.
g. The covering device (61b, 61d) comprises a longitudinal bore for accommodating therein at least one functional element (50a, 50b, 50c, 50d).
h. The covering device (61b, 61d) is configured as a sleeve, at least in sections.
i. The covering device (61b, 61d) is, at least in sections, pushed-onto, put-onto or screwed onto a proximal section of the guide element (40a, 40b, 40c, 40d).
j. The covering device (61b, 61d) is adapted to be inserted into a blood vessel.
k. The covering device (61b, 61d) comprises an accommodation unit having arranged therein the at least two functional elements (50a, 50b, 50d) such that they can be guided out.
l. The covering device (61b, 61d) is arranged movably along a longitudinal orientation relative to the functional elements (50a, 50b, 50c, 50d) and/or relative to the stabilization element (60a, 60b, 60d).
m. The covering device (61b) comprises a distal end (66b) and a proximal end (64b), wherein a thread, in particular a Luer-Lock thread, is formed on the distal end (66b) for connecting indwelling cannulas.
n. The covering device (61b, 61d) comprises homopolymers, copolymers, biopolymers, chemically modified polymers, and/or synthetic polymers.
o. The covering device (61b, 61d) has a rounded end to protect from injuries.
p. The covering device (61d) is configured for covering the joint between at least one of the functional elements (50d) and the guide element (40d).

5. The detection device (1a, 1b, 1c, 1d) according to claim 1, **characterized in that** at least one of the functional elements ((50a, 50b, 50c, 50d) satisfies at least one of the following requirements:
a. The functional element (50a, 50b, 50c, 50d) is at least partially made of metal, preferably high-grade steel, medical high-grade steel, or titanium; of glass, preferably glass fiber; of plastics, preferably foamed plastic, a polymer, preferably polyethylene, polypropylene, polyurethane, polytetrafluoroethylene, a plastic based on organic polymers, or a combination of these materials.
b. The functional element (50c) is fixed to the distal end (44c) of the guide element (40c), in particular to a cross-sectional surface of the distal end (44c).
c. The functional element (50c) is fixed to the outer periphery of the guide element (40c), the length of the functional element (50c) being preferably longer than the distance of the joint at the outer periphery of the guide element (40c) from the distal end (44c) of the guide element (40c).
d. The functional element (50c) is connected, at its distal end, to at least one further functional element (50c), preferably to at least two further functional elements (50c), preferably by a material bond.
e. The functional element (50d) is fixed to a cross-sectional surface of the distal end (44d), in particular welded to the cross-sectional surface of the distal end (44d).
f. The functional element (50d) is connected, at its distal end, to at least one further functional element (50d), preferably to at least two further functional elements (50d), in particular by a material bond, in particular welded.
g. The functional element (50a, 50b) is adapted to be non-destructively detached from the at least one other functional element (50a, 50b) and/or non-destructively removed from the guide element (40a, 40b).
h. The functional element (50c, 50d) is adapted to be cut off or torn off the guide element (40c, 40d).
i. The functional element (50a, 50b) comprises an internal thread on its inner peripheral surface (52a).
j. The functional element (50a, 50b) encloses at least one section of the guide element (40a, 40b).
k. The functional element (50a, 50b) is arranged on the guide element (40a, 40b) between a distal end (44a, 44b) and a proximal end (46a, 46b) of the latter.
l. The functional element (50a, 50b, 50c) comprises detection receptors (12) which differ from the detection receptors (12) of the respective other functional element (50a, 50b, 50c).
m. The functional element (50a, 50b, 50c) comprises a combination of detection receptors (12) which differs from the combination of detection receptors (12) of the respective other functional element (50a, 50b, 50c).
n. The functional element (50a, 50b) comprises at least one section having an outer diameter which corresponds or substantially corresponds to the outer diameter of the distal section (44a, 44b) of the guide element (40a, 40b).
o. The functional element (50a, 50b, 50c) comprises at least one section having an outer diameter which is larger or smaller than the distal section (44a, 44b) of the guide element (40a, 40b), in particular 0.01 mm to 0.1 mm smaller than the distal section (44a, 44b) of the guide element (40a, 40b).
p. The functional element (50a, 50b) is configured with elevations, indentations and/or branches.
q. The functional element (50a, 50b) comprises a coating (22) of metal, preferably of a metal of the 10^{th} or 11^{th} group of the periodic system of elements, preferably of nickel, copper, palladium, silver, platinum and/or gold.

6. The detection device (1a, 1b, 1c, 1d) according to at least one of the preceding claims, **characterized in that** the functional surface (10) of at least one functional element (50a, 50b, 50c, 50d) satisfies at least one of the following requirements:
a. The functional surface (10) comprises a three-dimensional structure with functional sections (11) facing each other and defining at least one space (13) through which a sample liquid can flow, the space (13) having preferably the shape of a channel, at least in sections, wherein preferably several spaces preferably form a branched network of channels.
b. The functional surface (10) is structured three-dimensionally on a macroscopic and/or microscopic scale.
c. The functional surface (10) is configured with elevations, indentations, and/or branches.
d. The functional surface (10) comprises, at least partially, a spiral, screw-shaped, worm-shaped, undulated, helical, filamentous, brush-like, comb-like, net-like, porous, spongy or similar structure.

7. The detection device (1a, 1b, 1c, 1d) according to at least one of the preceding claims, **characterized in that** the detection receptors (12) comprise antibodies, antibody fragments, amino acid structures, nucleic acid structures, inorganic materials and/or synthetic structures with a specific affinity to cellular surfaces, preferably monoclonal antibodies of murine origin, chimeric antibodies or humanized antibodies, preferably HLA-G and/or EpCAM antibodies.

8. The detection device (1a, 1b, 1c, 1d) according to at least one of the preceding claims, **characterized in that** at least one of the functional elements (50a, 50b) comprises a biocompatible polymer (3), which preferably satisfies at least one of the following requirements:
a. The biocompatible polymer (3) is configured as a coherent polymer layer.
b. The biocompatible polymer (3) has a three-dimensional, preferably filamentous and/or porous structure.
c. The biocompatible polymer (3) has a three-dimensional, preferably filamentous and/or porous surface.
d. The biocompatible polymer (3) has a carbonaceous, branched molecular structure.
e. The biocompatible polymer (3) is preferably actively connected to the functional element (50a, 50b, 50c, 50d) via functional groups, preferably by a chemical bond, particularly preferred by a covalent bond.
f. The biocompatible polymer (3) comprises functional groups, preferably carboxylic groups, wherein the functional groups preferably comprise an uncompensated molecular charge by chemical activation, wherein the functional groups are preferably adjusted to the detection receptors (12).
g. The biocompatible polymer (3) comprises hydrophilic properties and is preferably a hydrogel.
h. The biocompatible polymer (3) comprises chemically and/or enzymatically cleavable groups.
i. The biocompatible polymer (3) comprises saturated groups of atoms and covalently bound ligands and receptors.
j. The biocompatible polymer (3) is arranged in a cavity of the functional element (50a, 50b).
k. The biocompatible polymer (3) is cross-linked.
l. The biocompatible polymer (3) comprises and/or forms the functional surface (10).

9. The detection device (1a, 1b, 1c, 1d) according to at least one of the preceding claims, **characterized in that** the at least one functional surface (10) is coated with a protective layer (4), said protective layer (4) satisfying preferably at least one of the following requirements:
a. The protective layer (4) is soluble in liquids, in particular in body liquids, preferably in blood.
b. The protective layer (4) is biocompatible.
c. The protective layer (4) is organically crystalline and comprises at least one of the following components: alginates, preferably highly purified alginates, polyethylene glycols, cyclic and non-cyclic oligosaccharides, polysaccharides, antioxidative amino acids, proteins or vitamins.

10. The use of a detection device (1a, 1b, 1c, 1d) according to at least one of the preceding claims for in vitro enrichment of samples.

## Revendications

1. Dispositif de détection (1a, 1b, 1c, 1d) destiné à l'enrichissement de matériel échantillon in vivo et/ou in vitro, comportant au moins un élément de guidage (40a, 40b, 40c, 40d) destiné au guidage d'au moins un élément fonctionnel (40a, 40b, 40c, 40d) et comportant au moins deux éléments fonctionnels (50a, 50b, 50c, 50d) disposés sur l'élément de guidage (40a, 40b, 40c, 40d), sur chacun desquels est configurée une surface fonctionnelle (10) munie de récepteurs de détection (12), les éléments fonctionnels (50a, 50b, 50c, 50d) étant conçus pour être détachables les uns des autres et/ou étant individuellement détachables de l'élément de guidage (40a, 40b, 40c, 40d), **caractérisé en ce qu'**au moins un élément fonctionnel (50a, 50b) conçu sous forme de segment annulaire est vissé sur l'élément de guidage (40a, 40b) et/ou **en ce qu'**au moins un élément fonctionnel (50c, 50d) conçu sous forme de fil ou fil métallique flexible est fixé par adhérence à l'extrémité distale (44c, 44d) de l'élément de guidage (40c, 40d).

2. Dispositif de détection (1a, 1b, 1c, 1d) selon au moins l'une quelconque des revendications précédentes, **caractérisé en ce que** l'élément de guidage (40a, 40b, 40c, 40d) satisfait à au moins l'une des exigences suivantes :
a. L'élément de guidage (40a, 40b, 40c, 40d) est au moins par tronçons conçu sous forme de fil métallique.
b. L'élément de guidage (40a, 40b, 40c, 40d) est au moins par tronçons conçu pour être élastique à la manière d'un ressort.
c. L'élément de guidage (40a, 40b, 40c, 40d) est au moins par tronçons conçu sous forme de fil métallique de guidage médical flexible.
d. L'élément de guidage (40a, 40b, 40c) est au moins par tronçons conçu sous forme de fil.
e. L'élément de guidage (40a, 40b, 40c) est au moins par tronçons conçu sous forme de fil en matière plastique flexible.
f. L'élément de guidage (40a, 40b, 40c) est au moins par tronçons conçu sous forme de cathéter.
g. L'élément de guidage (40a, 40b) présente un tronçon de réception destiné à la réception d'au moins un élément fonctionnel (50a, 50b).
h. L'élément de guidage (40a, 40b) présente une extrémité distale (44a, 44b) ainsi qu'une extrémité proximale (46a, 46b), un tronçon de réception destiné à la réception d'au moins un élément fonctionnel (50a, 50b) étant conçu entre l'extrémité distale et l'extrémité proximale.
i. L'élément de guidage (40a, 40b, 40c) est au moins par tronçons conçu sous forme de vis.
j. L'élément de guidage (40a) comporte à sa périphérie externe (42a) au moins par tronçons un filetage extérieur.
k. L'élément de guidage (40a, 40b, 40c, 40d) présente une extrémité distale (44a, 44b, 44c, 44d) ainsi qu'une extrémité proximale (46a, 46b, 46c, 46d), l'extrémité distale (44a, 44b, 44c, 44d) pouvant être introduite dans un vaisseau sanguin.
l. L'élément de guidage (40a, 40b) présente une extrémité distale (44a, 44b) ainsi qu'une extrémité proximale (46a, 46b), l'extrémité distale (44a, 44b) étant conçue épaissie.
m. L'élément de guidage (40a) présente une extrémité distale (44a) ainsi qu'une extrémité proximale (46a), l'extrémité proximale (46a) étant raccordée à un élément de stabilisation (60a).
n. L'élément de guidage (40a) est vissé dans un filetage intérieur de l'élément de stabilisation (60a).
o. L'élément de guidage (40a, 40b, 40c, 40d) est fabriqué à partir d'un matériau métallique et/ou non métallique.
p. L'élément de guidage (40a, 40b, 40c, 40d) présente à sa partie distale une surface fonctionnelle (10) munie de récepteurs de détection (12).

3. Dispositif de détection (1a, 1b, 1c, 1d) selon au moins l'une quelconque des revendications précédentes, **caractérisé en ce que** l'élément de guidage (40a, 40b, 40c, 40d) est raccordé à un élément de stabilisation (60a, 60b, 60d) destiné à la stabilisation dudit au moins un élément de guidage (40a, 40b, 40c, 40d), qui de préférence satisfait à au moins l'une des exigences suivantes :
a. L'élément de stabilisation (60a, 60b, 60d) est conçu pour stabiliser au moins par tronçons L'élément de guidage (40a, 40b, 40d).
b. L'élément de stabilisation (60a, 60b, 60d) est fabriqué à partir de matière plastique ou de métal.
c. L'élément de stabilisation (60a, 60b, 60d) est raccordé à une extrémité proximale (46a, 46b, 46d) ou à une partie terminale proximale (46a, 46b, 46d) de l'élément de guidage (40a, 40b, 40d).
d. L'élément de stabilisation (60a, 60b, 60d) est raccordé de façon détachable à l'élément de guidage (40a, 40b, 40d).
e. L'élément de stabilisation (60a, 60b, 60d) est raccordé par emboîtement, mécaniquement et/ou par adhérence à l'élément de guidage (40a, 40b, 40d).
f. L'élément de stabilisation (60a, 60b, 60d) est collé ou soudé à l'élément de guidage (40a, 40b, 40d).
g. L'élément de stabilisation (60a, 60b, 60d) a au moins par tronçons une configuration cylindrique.
h. L'élément de stabilisation (60a, 60b, 60d) est au moins par tronçons conçu sous forme de manchon (60a, 61b, 61d).
i. L'élément de stabilisation (60a, 60b, 60d) comporte au moins par tronçons un filetage intérieur.
j. L'élément de stabilisation (60a, 60b) est au moins par tronçons emboîté, monté ou vissé sur une partie proximale de l'élément de guidage (40a, 40b).
k. L'élément de stabilisation (60d) est au moins par tronçons emboîté, monté ou vissé sur une partie distale (44d) de l'élément de guidage (40d).
l. L'élément de stabilisation (60a, 60b, 60d) guide au moins par tronçons l'élément de guidage (40a, 40b, 40d).
m. L'élément de stabilisation (60a, 60b, 60d) guide de façon mobile l'élément de guidage (40a, 40b, 40d).
n. L'élément de stabilisation (60b, 60d) est conçu sous forme de dispositif de recouvrement pour au moins un élément fonctionnel (50b, 50d).
o. L'élément de stabilisation (60b, 60d) comporte un logement dans lequel sont disposés lesdits au moins deux éléments fonctionnels (50b, 50d).
p. L'élément de stabilisation (60b, 60d) peut être introduit dans un vaisseau sanguin.
q. L'élément de stabilisation (60b, 60d) comporte un logement dans lequel lesdits au moins deux éléments fonctionnels (50b, 50d) sont disposés de manière à pouvoir en être retirés.
r. L'élément de stabilisation (60b) présente une extrémité distale (66b) et une extrémité proximale (64b), à l'extrémité distale (66b) étant formé un filetage, en particulier un filetage Luer-Lock, destiné au raccordement de canules permanentes.
s. L'élément de stabilisation (60a, 60b) comporte un dispositif de poussée-traction (68b), au moyen duquel lesdits au moins deux éléments fonctionnels (50b) peuvent être extraits du manchon (61b) et réintroduits dans celui-ci.
t. L'élément de stabilisation (60a, 60b) présente au moins un tronçon dont le diamètre externe correspond ou correspond essentiellement au diamètre externe du tronçon distal (44a, 44b) de l'élément de guidage (40a, 40b).
u. L'élément de stabilisation (60a, 60b) est fabriqué avec l'élément de guidage (40a, 40b) dans un processus de fabrication.
v. L'élément de stabilisation (60a, 60b, 60d) présente une extrémité arrondie en tant que protection contre les blessures.
w. L'élément de stabilisation (60d) est destiné à la stabilisation du point de raccordement entre au moins l'un des éléments fonctionnels (50d) ainsi que l'élément de guidage (40d).

4. Dispositif de détection (1a, 1b, 1c, 1d) selon au moins l'une quelconque des revendications précédentes, **caractérisé en ce qu'**est prévu un dispositif de recouvrement (61b, 61d) qui satisfait à au moins l'une des exigences suivantes :
a. Le dispositif de recouvrement (61b, 61d) est conçu pour le recouvrement d'au moins l'un des éléments fonctionnels (50a, 50b, 50c, 50d).
b. Le dispositif de recouvrement (61b, 61d) est un composant de l'élément de stabilisation (60a, 60b, 60d).
c. Le dispositif de recouvrement (61b, 61d) est conçu sous forme de composant distinct de l'élément de stabilisation (60a, 60b, 60d).
d. Le dispositif de recouvrement (61b, 61d) est conçu pour stabiliser au moins par tronçons l'élément de guidage (40a, 40b, 40c, 40d).
e. Le dispositif de recouvrement (61b, 61d) est fabriqué à partir de matière plastique ou de métal.
f. Le dispositif de recouvrement (61b, 61d) a au moins par tronçons une configuration cylindrique.
g. Le dispositif de recouvrement (61b, 61d) présente un alésage longitudinal, destiné à recevoir au moins un élément fonctionnel (50a, 50b, 50c, 50d).
h. Le dispositif de recouvrement (61b, 61d) est au moins par tronçons conçu sous forme de manchon.
i. Le dispositif de recouvrement (61b, 61d) est au moins par tronçons emboîté, monté ou vissé sur un tronçon proximal de l'élément de guidage (40a, 40b, 40c, 40d).
j. Le dispositif de recouvrement (61b, 61d) peut être introduit dans un vaisseau sanguin.
k. Le dispositif de recouvrement (61b, 61d) comporte un logement dans lequel sont disposés de manière à pouvoir en être retirés lesdits au moins deux éléments fonctionnels (50a, 50b, 50d).
l. Le dispositif de recouvrement (61b, 61d) est disposé de façon mobile le long d'une orientation longitudinale par rapport aux éléments fonctionnels (50a, 50b, 50c, 50d) et/ou par rapport à l'élément de stabilisation (60a, 60b, 60d).
m. Le dispositif de recouvrement (61b) présente une extrémité distale (66b) et une extrémité proximale (64b), à l'extrémité distale (66b) étant formé un filetage, en particulier un filetage Luer-Lock, destiné au raccordement de canules permanentes.
n. Le dispositif de recouvrement (61b, 61d) comporte des homopolymères, des copolymères, des biopolymères, des polymères modifiés chimiquement et/ou des polymères synthétiques.
o. Le dispositif de recouvrement (61b, 61d) présente une extrémité arrondie en tant que protection contre les blessures.
p. Le dispositif de recouvrement (61d) est destiné au recouvrement du point de raccordement entre au moins l'un des éléments fonctionnels (50d) ainsi que l'élément de guidage (40d).

5. Dispositif de détection (1a, 1b, 1c, 1d) selon la revendication 1, **caractérisé en ce qu**'au moins l'un des éléments fonctionnels (45a, 50b, 50c, 50d) satisfait à au moins l'une des exigences suivantes :
a. L'élément fonctionnel (50a, 50b, 50c, 50d) est au moins en partie fabriqué à partir de métal, de préférence acier inoxydable, acier inoxydable médical ou titane ; à partir de verre, de préférence fibre de verre, à partir de matière plastique, de préférence d'une matière plastique expansée, d"un polymère, de préférence de polyéthylène, polypropylène, polyuréthane, polytétrafluoroéthylène, d'une matière plastique à base de polymères organiques, ou d'une combinaison de ces matériaux.
b. L'élément fonctionnel (50c) est fixé à l'extrémité distale (44c) de l'élément de guidage (40c), en particulier à une surface de la section transversale de l'extrémité distale (44c).
c. L'élément fonctionnel (50c) est fixé à la périphérie externe de l'élément de guidage (40c), la longueur de l'élément fonctionnel (50c) étant de préférence supérieure à la distance entre le point de raccordement à la périphérie externe de l'élément de guidage (40c) et l'extrémité distale (44c) de l'élément de guidage (40c).
d. L'élément fonctionnel (50c) est à son extrémité distale lié, de préférence lié par adhérence, à au moins au un autre élément fonctionnel (50c), de préférence à au moins deux autres éléments fonctionnels (50c).
e. L'élément fonctionnel (50d) est fixé à une surface de la section transversale de l'extrémité distale (44d), en particulier soudé à la surface de la section transversale de l'extrémité distale (44d).
f. L'élément fonctionnel (50d) est à son extrémité distale lié, en particulier lié par adhérence, en particulier soudé, à au moins un autre élément fonctionnel (50d), de préférence à au moins deux autres éléments fonctionnels (50d).
g. L'élément fonctionnel (50a, 50b) est détachable sans dommage d'au moins un autre élément fonctionnel (50a, 50b) et/ou séparable sans dommage de l'élément de guidage (40a, 40b).
h. L'élément fonctionnel (50c, 50d) est séparable de l'élément de guidage (40c, 40d) par découpage ou arrachage.
i. L'élément fonctionnel (50a) comprend un filetage intérieur sur sa surface périphérique interne (52a).
j. L'élément fonctionnel (50a, 50b) entoure au moins un tronçon de l'élément de guidage (40a, 40b).
k. L'élément fonctionnel (50a, 50b) est disposé sur l'élément de guidage (40a, 40b) entre une extrémité distale (44a, 44b) et une extrémité proximale (46a, 46b) de ce dernier.
l. L'élément fonctionnel (50a, 50b, 50c) comporte des récepteurs de détection (12) qui se distinguent des récepteurs de détection (12) de l'autre élément fonctionnel (50a, 50b, 50c) respectif.
m. L'élément fonctionnel (50a, 50b, 50c) comporte une combinaison de récepteurs de détection (12) qui se distinguent de la combinaison de récepteurs de détection (12) de l'autre élément fonctionnel (50a, 50b, 50c) respectif.
n. L'élément fonctionnel (50a, 50b) présente au moins un tronçon ayant un diamètre externe qui correspond ou correspond essentiellement au diamètre externe du tronçon distal (44a, 44b) de l'élément de guidage (40a, 40b).
o. L'élément fonctionnel (50a, 50b, 50c) présente au moins un tronçon ayant un diamètre externe qui est supérieur ou inférieur à celui du tronçon distal (44a, 44b) de l'élément de guidage (40a, 40b), en particulier inférieur de 0,01 mm à 0,1 mm à celui du tronçon distal (44a, 44b) de l'élément de guidage (40a, 40b).
p. L'élément fonctionnel (50a, 50b) est configuré avec des saillies, des évidements et/ou des ramifications.
q. L'élément fonctionnel (50a, 50b) comprend un revêtement (22) à base de métal, de préférence à base d'un métal du 10^{e} ou 11^{e} groupe du système périodique des éléments, de préférence à base de nickel, cuivre, palladium, d'argent, de platine et/ou d'or.

6. Dispositif de détection (1a, 1b, 1c, 1d) selon au moins l'une quelconque des revendications précédentes, **caractérisé en ce que** la surface fonctionnelle (10) d'au moins un élément fonctionnel (50a, 50b, 50c, 50d) satisfait à au moins l'une des exigences suivantes :
a. La surface fonctionnelle (10) présente une structure tridimensionnelle avec des parties fonctionnelles (11) se faisant face, qui forment au moins un espace intermédiaire (13) susceptible d'être chargé d'un liquide échantillon, l'espace intermédiaire (13) étant de préférence configuré au moins par endroits en forme de canal, de préférence plusieurs espaces intermédiaires formant de préférence un réseau ramifié de canaux.
b. La surface fonctionnelle (10) présente une structure tridimensionnelle à l'échelle macroscopique et/ou à l'échelle microscopique.
c. La surface fonctionnelle (10) est configurée avec des saillies, des évidements et/ou des ramifications.
d. La surface fonctionnelle (10) comprend au moins en partie une structure spiralée, en forme de vis, en colimaçon, ondulée, hélicoïdale, filamenteuse, en brosse, en peigne, en forme de filet, poreuse, spongieuse ou similaire.

7. Dispositif de détection (1a, 1b, 1c, 1d) selon au moins l'une quelconque des revendications précédentes, **caractérisé en ce que** les récepteurs de détection (12) comprennent des anticorps, des fragments d'anticorps, des structures d'acides aminés, des structures d'acide nucléique, des matières inorganiques et/ou des structures synthétiques à affinité spécifique pour les surfaces de cellules, de préférence des anticorps monoclonaux d'origine murine, des anticorps chimériques ou des anticorps humanisés, de préférence des anticorps HLA-G et/ou EpCAM.

8. Dispositif de détection (1a, 1b, 1c, 1d) selon au moins l'une quelconque des revendications précédentes, **caractérisé en ce qu**'au moins l'un des éléments fonctionnels (50a, 50b) comporte un polymère biocompatible (3) qui de préférence satisfait à au moins l'une des exigences suivantes :
a. Le polymère biocompatible (3) est sous forme de couche de polymère continue.
b. Le polymère biocompatible (3) présente une structure tridimensionnelle, de préférence filamenteuse et/ou poreuse.
c. Le polymère biocompatible (3) présente une surface tridimensionnelle, de préférence filamenteuse et/ou poreuse.
d. Le polymère biocompatible (3) présente une structure moléculaire ramifiée, contenant du carbone.
e. Le polymère biocompatible (3) est de préférence fonctionnellement lié par des groupes fonctionnels à l'élément fonctionnel (50a, 50b, 50c, 50d), de préférence par liaison chimique, de façon particulièrement préférée par liaison covalente.
f. Le polymère biocompatible (3) comprend des groupes fonctionnels, de préférence des groupes carboxy, les groupes fonctionnels présentant de préférence par activation chimique une charge moléculaire non neutralisée, les groupes fonctionnels étant de préférence adaptés aux récepteurs de détection (12).
g. Le polymère biocompatible (3) présente des propriétés hydrophiles et est de préférence un hydrogel.
h. Le polymère biocompatible (3) comprend des groupes séparables par voie chimique et/ou voie enzymatique.
i. Le polymère biocompatible (3) comprend des groupes d'atomes saturés et des récepteurs et ligands liés par liaison covalente.
j. Le polymère biocompatible (3) est disposé dans une cavité de l'élément fonctionnel (50a, 50b).
k. Le polymère biocompatible (3) est réticulé.
l. Le polymère biocompatible (3) comprend et/ou constitue la surface fonctionnelle (10).

9. Dispositif de détection (1a, 1b, 1c, 1d) selon au moins l'une quelconque des revendications précédentes, **caractérisé en ce que** ladite au moins une surface fonctionnelle (10) est revêtue d'une couche protectrice (4), la couche protectrice (4) de préférence satisfaisant à au moins l'une des exigences suivantes :
a. La couche protectrice (4) est soluble dans des liquides, en particulier dans des liquides corporels, de préférence dans le sang.
b. La couche protectrice (4) est biocompatible.
c. La couche protectrice (4) est organiquement cristalline et comprend au moins l'un des composants suivants : alginates, de préférence alginates très purs, polyéthylèneglycols, oligosaccharides cycliques et oligosaccharides non cycliques, polysaccharides, acides aminés antioxydants, protéines ou vitamines.

10. Utilisation d'un dispositif de détection (1a, 1b, 1c, 1d) selon au moins l'une quelconque des revendications précédentes, pour l'enrichissement in vitro d'échantillons.
